# EUROPEAN PATENT APPLICATION

(11) **EP 4 434 968 A1**
(43) Date of publication of application: **25.09.2024**
(21) Application number: 22894919.4
(22) Date of filing: 17.11.2022
(51) Int. Cl.: C07D 209/88, C07D 231/12, C07D 241/04, C07D 487/04, C07D 495/04, A61K 31/403, A61K 31/4155, A61K 31/495, A61P 35/00

(54) **TETRAHYDROCARBAZOLE COMPOUND, AND PHARMACEUTICAL COMPOSITION AND USE THEREOF**

(30) Priority: 19.11.2021 CN 202111410807
(71) Applicant: Nain Biotech (Hangzhou) Co., Ltd., Hangzhou, Zhejiang 311112 (CN)
(72) Inventor: WANG, Yingjie, Hangzhou, Zhejiang 311112 (CN); KANG, Bo, Hangzhou, Zhejiang 311112 (CN); JIA, Wei, Hangzhou, Zhejiang 311112 (CN)
(74) Representative: Dehns
(86) International application number: PCT/CN2022/132643
(87) International publication number: WO 2023/088388

(57) **Abstract**

The present invention relates to a tetrahydrocarbazole compound, and a pharmaceutical composition and the use thereof. The tetrahydrocarbazole compound is a compound as represented by formula (I), an optical isomer or a pharmaceutically acceptable salt thereof. The provided tetrahydrocarbazole compound, on one hand, can jointly inhibit OCT4 at the transcriptional and functional levels by means of targeting OCT4 and JAK to promote CSC differentiation, and on the other hand, inhibits differentiated tumor cells by means of inhibiting a JAK/STAT pathway. The compound provided in the present invention acts on two drug targets of OCT4 and JAK/STAT in tumor cells, therapy synergistically inhibiting tumor proliferation and metastasis by means of a dual-targeting effect, and can be used for preparing a drug for treating and/or preventing of cancers.

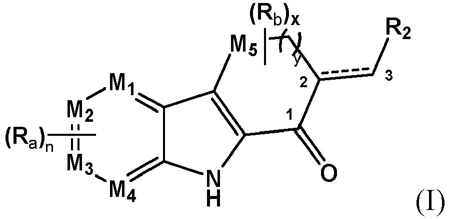

## Description

### TECHNICAL FIELD

The present invention relates to the field of pharmaceutical technology, specifically to a tetrahydrocarbazole compound, and pharmaceutical composition and use thereof.

### BACKGROUND TECHNOLOGY

Cancer stem cells (CSC) are a small subgroup of tumor cells, accounting for only a small fraction (usually 1% or less) of tumor tissue, and they possess several key characteristics similar to normal stem cells. The first characteristic is self-renewal, CSCs maintain the ability to undergo cell division continuously and differentiate into various types of tumor cells. The second characteristic is the ability to undergo asymmetric division, i.e., dividing to produce a daughter CSC with the same characteristics as the mother CSC, while a differentiated daughter cell is also produced, those daughter cells rapidly proliferates to occupy a large portion of the tumor volume, and eventually, the CSCs and their differentiated daughter cells together constitute tumor tissue with the abilities of self-renewal and rapid proliferation (Clevers H. The cancer stem cell: Premises, promises and challenges. Nat Med 011,17:313-319). This means that the contribution of CSCs to the long-term survival of tumors is much greater than that of their daughter differentiated tumor cells.

In recent years, the transformation between adult stem cells (ASC) and CSCs has also received widespread attention. Mutations in normal ASC cause dysregulation of self-renewal and induce cell to transform to CSC. These mutated CSCs exhibit typical malignant tumor characteristics such as stronger ability of proliferation, anti-apoptosis and immune escape, which are important reasons for tumor resistance and recurrence.

CSCs have been reported to have extensive metastatic potential, and regenerate tumors and generate disseminated metastatic tumors (Wang YJ, Herlyn M. The emerging roles of OCT4 in tumor-initiating cells. Am J Physical Cell Physical 2015, 309: C709-718). Currently, almost all drugs and radiation treatments can only kill or inhibit rapidly proliferating differentiated tumor cells, but cannot effectively remove or inhibit CSCs. Since the CSCs and differentiated tumor cells can transform into each other under specific conditions, only combination therapies that simultaneously target the inhibition or killing of CSCs and differentiated tumor cells, and completely blocks the bidirectional transformation between them, may make it possible to eradicate the tumor.

So far, there have been no drugs specifically targeting CSCs on the market, and a series of candidate drugs (such as Notch, Wnt inhibitors) that can promote CSCs differentiation or inhibit their proliferation are at various stages of clinical trials (Maurort C et al. Differentiation of cancer stem cells by using synthetic small molecules: toward new therapeutic strategies against therapyresistance ChemMedChem 2021, 16:14-29). CSCs exhibit resistance to conventional radiotherapy, chemotherapy, and molecular targeted therapy drugs, making specifically targeting CSCs become an important strategy for tumor treatment and anti-tumor resistance research.

The core stem transcription factor OCT4 is a member of the POU transcription factor family, and its coding gene POU5F1 has multiple transcription start sites, which can transcribe different mRNA isoforms and then translate them into various OCT4 subtype proteins. OCT4A (commonly referred to as OCT4), as a classical transcription factor, has a POU domain that can specifically recognize and bind to the octamer motifs (ATGC (A/T) AAT) of numerous target gene promoter regions. The OCT4 protein regulates the transcription of hundreds of downstream target genes through collaboration with interacting proteins such as SOX2, and these target genes can usually be divided into two categories: one is the stem factors that positively activate its transcription by OCT4/SOX2, such as NANOG genes, and the other is the specific genes which are differentiated from various germ layers and negatively inhibit its transcription by OCT4/SOX2. Therefore, OCT4/SOX2 maintains self-renewal and pluripotency of pluripotent Stem Cells (PSC) by simultaneously promoting stem gene expression and shutting down germ gene expression, and is at the center of the PSC homeostasis regulatory network (Jerabek S et al. OCT4: Dynamic DNA binding pioneers stem cell pluripotency. Biochim Biophys Acta 2014, 1839:138-154).

The expression of OCT4 is closed in normal differentiated tissues, but it is slightly expressed in various solid tumor tissues and their cell lines, such as liver cancer, lung cancer, breast cancer, pancreatic cancer, gastric cancer, cervical cancer, ovarian cancer, head and neck cancer, etc. And its expression is significantly up-regulated under the stimulation of some growth factors and hypoxia conditions, which further suggests the plasticity of OCT4 expression in these cells. The expression of OCT4 in tumor tissues and cells exhibits heterogeneity: relative to most differentiated tumor cells, OCT4 is mainly expressed in a small fraction of CSCs, and thus has gradually been recognized as a biomarker of CSCs in recent years (Wang YJ, Herlyn M. The emerging roles of OCT4 in tumor initiating cells. Am J Physical Cell Physiol 2015, 309: C709-718).

The research conducted by the applicant's laboratory has shown that knocking out the OCT4 gene with CRISPR/Cas9 in tumor cells such as hepatocellular and cervical carcinomas cell significantly inhibits the proliferation and metastasis of CSCs, promotes its differentiation and apoptosis, thus confirming that OCT4 is a potential drug target for anti-tumor cells, especially for CSCs. (Zhou Y et al. Endogenous authentic OCT4A proteins directly regulate FOS/AP-1 transcription in somatic cancer cells. Cell Death Dis. 2018, 9:585; Ye C et al. Multiple novel hepatocellular carcinoma signature genes are commonly controlled by the master pluripotency factor OCT4.Cell Oncol (Dordr).2020 , 43:279-295). Although OCT4 plays a crucial role in CSCs self-renewal and survival, tumor metastasis, and tumor resistance, small molecule compounds that can specifically target and inhibit OCT4 have hardly been reported.

It is known that compounds such as retinoic acid can promote PSC differentiation through inhibiting transcription of OCT4 gene, but these compounds have multiple targets and mechanisms of action, and they are not specifically targeted to inhibit OCT4. KRIBB53, the only small molecule compound reported so far that can specifically target the OCT4 protein, downregulates the level of OCT4 protein in cells by promoting ubiquitination degradation of OCT4 protein, but the specific site and mechanism of action are still unclear. Moreover, KRIB53 has a relatively weak efficacy with an IC₅₀ of 10-20 µM in inhibiting the proliferation of two types of PSCs, NCCIT and TERA1.(Jung J et al. KRIBB53 binds to OCT4 and enhances its degradation through the proteasome, causing apoptotic cell death of OCT4-positive testicular germ cell tumors. Carcinogenesis.2018, 39:838-849).

In addition, due to the low average expression level of OCT4 in tumor cells, limited drug efficacy, and negative feedback compensatory mechanisms in tumor signaling pathways, it is difficult to completely inhibit the role of OCT4 in tumor cells by targeting the expression and transcription factor function of OCT4 alone. Among the regulatory pathway of OCT4, the JAK/STAT pathway not only directly regulates the transcription of OCT4 and NANOG to maintain the multifunctionality of CSC, but also becomes an important therapeutic target for differentiated tumor cells because of its high activation in various tumors and its involvement in malignant transformation of tumor cells. Currently, the molecular targeted drugs against the JAK/STAT pathway that commonly used in clinic are mainly used to treat autoimmune diseases and bone marrow fibrosis, and their efficacy in targeted treatment for tumors remains to be determined. Therefore, the development of JAK/STAT inhibitors with tumor suppressive effects has good application prospects.

### SUMMARY OF THE INVENTION

In view of all or part of the shortcomings of the prior art mentioned above, the present invention provides a class of tetrahydrocarbazole compound, and pharmaceutical composition and use thereof. On the one hand, the provided tetrahydrocarbazole compounds can jointly inhibit OCT4 at the transcriptional and functional levels by means of targeting OCT4 and JAK to promote CSCs differentiation, and on the other hand, inhibit differentiated tumor cells by means of inhibiting the JAK/STAT pathway. The compounds provided in the present invention act on two drug targets of OCT4 and JAK/STAT in tumor cells, and synergistically inhibit tumor proliferation and metastasis by means of a dual-targeting effect,, and can be used for preparing drug for treating and/or preventing of cancers.

The first aspect of the present invention provided is a compound as shown in formula (I), an optical isomer or a pharmaceutically acceptable salt thereof: 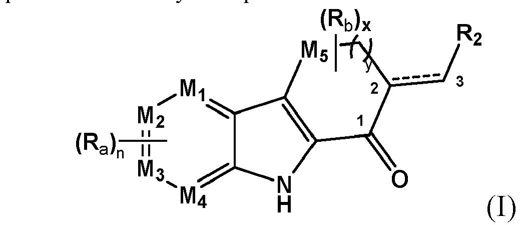 wherein, 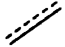
is a single or double bond;
R₂ is selected from the group consisting of phenyl, 5-7-membered heteroaryl and 8-12-membered fused heteroaryl, which is unsubstituted or substituted by one or more R_{c};
M₁, M₂, M₃ and M₄ are independently selected from the group consisting of CH, CD and N; and when M₁, M₂, M₃ or M₄ is CH, the hydrogen atom on the CH can be optionally substituted by Rₐ;
M₅ is selected from the group consisting of CH₂, CHD, CD₂, NH, S, S(=O), S(=O)₂, and S(=O)(=NH); and when M₅ is CH₂ or NH, the hydrogen atom on the CH₂ or NH can be optionally substituted by R_{b};
Rₐ is selected from the group consisting of hydrogen, deuterium, halogen, hydroxyl, carboxyl, amino, cyano, sulfonyl, C(O)NH₂, C₁₋₅ alkyl, C₂₋₅ alkenyl, C₂₋₅ alkynyl, C₁₋₅ alkoxy, C₁₋₅ haloalkyl, C₂₋₅ haloalkenyl, C₂₋₅ haloalkynyl, C₁₋₅ haloalkoxy, C₂₋₅ ester group, C₁₋₅ carbonyl, C(O)NH(C₁₋₅alkyl), C₃₋₈ saturated or partially unsaturated carbocyclyl, 3-10 membered saturated or partially unsaturated heterocyclyl, C₆₋₁₀ aryl, and 5-12 membered heteroaryl; and the alkyl, alkenyl, alkynyl, alkoxy, haloalkyl, haloalkenyl, haloalkynyl, haloalkoxy, ester group, carbonyl, carbocyclyl, heterocyclyl, aryl and heteroaryl are independently and optionally substituted by one or more substituents selected from the group consisting of halogen, deuterium, hydroxyl, carboxyl, amino, nitro, cyano, sulfonyl, C₁₋₃alkyl and C₁₋₃ amino;
R_{b} is selected from the group consisting of hydrogen, deuterium, halogen, hydroxyl, carboxyl, cyano, sulfonyl, C₁₋₅ alkyl, C₂₋₅ alkenyl, C₂₋₅alkynyl, C₁₋₅ alkoxy, C₁₋₅ haloalkyl, C₂₋₅ haloalkenyl, C₂₋₅ haloalkynyl, C₁₋₅ haloalkoxy, C₂₋₅ ester group, C₁₋₅ carbonyl, C₃₋₈ saturated or partially unsaturated carbocyclyl, 3-10 membered saturated or partially unsaturated heterocyclyl, C₆₋₁₀ aryl, and 5-12 membered heteroaryl; and the alkyl, alkenyl, alkynyl, alkoxy, haloalkyl, haloalkenyl, haloalkynyl, haloalkoxy, ester group, carbonyl, carbocyclyl, heterocyclyl, aryl and heteroaryl are independently and optionally substituted by one or more substituents selected from the group consisting of halogen, deuterium, hydroxyl, carboxyl, nitro, cyano, sulfonyl, C₁₋₃ alkyl and C₁₋₃ amino;
R_{c} is selected from the group consisting of hydrogen, deuterium, halogen, hydroxyl, carboxyl, amino, nitro, cyano, sulfonyl, C₁₋₅ alkyl, C₂₋₅ alkenyl, C₂₋₅ alkynyl,C₁₋₅ alkoxy, C₁₋₅ haloalkyl, C₂₋₅ haloalkenyl, C₂₋₅ haloalkynyl, C₁₋₅ haloalkoxy, C₁₋₅ amino, C₂₋₅ ester group, C₁₋₅ carbonyl, C₃₋₈ saturated or partially unsaturated carbocyclyl, 3-10 membered saturated or partially unsaturated heterocyclyl, C₆₋₁₀ aryl, and 5-12 membered heteroaryl; and the alkyl, alkenyl, alkynyl, alkoxy, haloalkyl, haloalkenyl, haloalkynyl, haloalkoxy, amino, ester group, carbonyl, carbocyclyl, heterocyclyl, aryl and heteroaryl are independently and optionally substituted by one or more substituents selected from the group consisting of halogen, deuterium, hydroxyl, carboxyl, amino, nitro, cyano, sulfonyl and C₁₋₃ alkyl;
N is 0, 1, 2 or 3;
X is 0, 1, 2 or 3; and
Y is 0, 1 or 2.

Preferably, the compound does not include compounds selected from the group consisting of

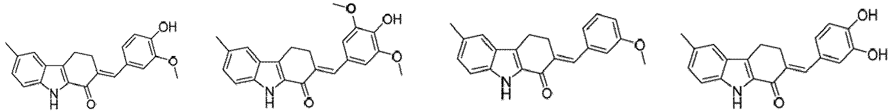

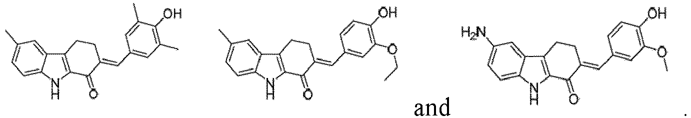

In some embodiments, the compound has a structure as shown in formula (II) or formula (III): 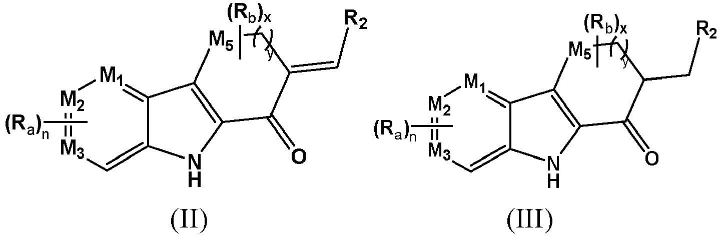 wherein, R₂, M₁, M₅, Rₐ, R_{b}, n, x and y are defined as described in claim 1.

In another preferred embodiment, the compound has a structure selected from the group consisting of: 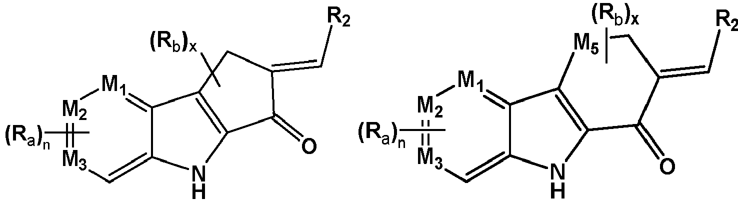 and

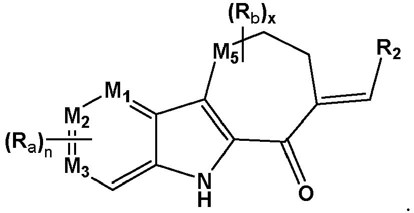

In another preferred embodiment, the compound has a structure selected from the group consisting of:

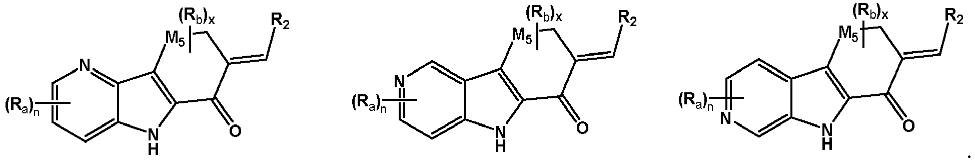

In some embodiments, R₂ is a unsubstituted or one or more R_{c} substituted phenyl, or a unsubstituted or one or more R_{c} substituted 5-10 membered heteroaryl;
wherein R_{c} is selected from the group consisting of hydrogen, halogen, hydroxyl, carboxyl, amino, nitro, cyano, sulfonyl, C₁₋₅ alkyl, C₂₋₅ alkenyl, C₂₋₅ alkynyl, C₁₋₅ alkoxy, C₁₋₅ haloalkyl, C₂₋₅ haloalkenyl, C₂₋₅ haloalkynyl, C₁₋₅ haloalkoxy, C₁₋₅ amino, and C₂₋₅ ester group.

In another preferred embodiment, R₂ is selected from the group consisting of phenyl,

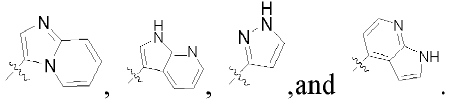

In some embodiments, R_{b} is selected from the group consisting of hydrogen, halogen, hydroxyl, carboxyl, cyano, sulfonyl, C₁₋₄ alkyl, C₁₋₄ alkoxy, C₁₋₄ haloalkyl, C₁₋₄ haloalkoxy, C₂₋₄ ester group and C₁₋₄ carbonyl; and the alkyl, alkoxy, haloalkyl, haloalkoxy, ester group and carbonyl are independently and optionally substituted by one or more substituents selected from the group consisting of halogen, hydroxyl, carboxyl, nitro, cyano, sulfonyl, C₁₋₃ alkyl and C₁₋₃ amino.

In some embodiments, Rₐ is selected from the group consisting of hydrogen, halogen, hydroxyl, carboxyl, cyano, C₁₋₄ alkyl, C₂₋₄ alkenyl, C₂₋₄ alkynyl, C₁₋₄ alkoxy, C₂₋₄ ester group, C₁₋₄ carbonyl, 3-8-membered saturated or partially unsaturated heterocyclyl, C₆₋₁₀ aryl, and 5-12membered heteroaryl; and the alkyl, alkenyl, alkynyl, alkoxy, ester group, carbonyl, heterocyclyl, aryl and heteroaryl are independently and optionally substituted by one or more substituents selected from the group consisting of halogen, hydroxyl, carboxyl, amino, nitro, cyano, sulfonyl, C₁₋₃ alkyl and C₁₋₃ amino.

The second aspect of the present invention provided is a compound shown in formula (IA), an optical isomer, or a pharmaceutically acceptable salt thereof: 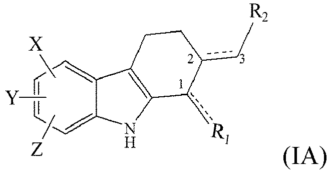 wherein,
R₁ is hydroxyl and is connected to the carbon atom at position 1 via a single bond, or R₁ is an oxygen atom or -N(CH₂)ₙOH and is connected to the carbon atom at position 1 via a double bond, wherein, n is 1 or 2;
the carbon atom at position 2 is connected to the carbon atom at position 3via a single or double bond; R₂ is selected from any one of the group consisting of unsubstituted or substituted monocyclyl or monoheterocyclyl, unsubstituted or substituted fused-cyclyl or fused heterocyclyl;
X, Y and Z are independently selected from any one of the group consisting of hydrogen, halogen, hydroxyl, carboxyl, amino, nitro, cyano, sulfonyl, C₁₋₅ alkyl, C₁₋₅ alkoxy, C₁₋₅ haloalkyl and C₁₋₅ haloalkoxy.

In some embodiments, in the compound shown in formula I, R₁ is an oxygen atom and is connected to the carbon atom at position1via a carbon-oxygen double bond; the carbon atom at position 2 is connected to the carbon atom at position 3 via a carbon-carbon double bond, and comprises the compound shown in formula (IIA): 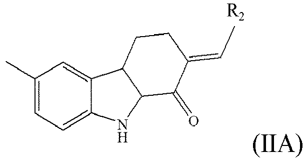 in formula IIA:
R₂ is selected from any one of group consisting of unsubstituted or substituted monocyclyl or monoheterocyclyl, unsubstituted or substituted fused-cyclyl or fused heterocyclyl; and the substituents on the monocyclyl, monoheterocyclyl, fused-cyclyl or fused heterocyclyl are selected from one or more group consisting of halogen, hydroxyl, carboxyl, amino, nitro, cyano, sulfonyl, C₁₋₅ alkyl, C₁₋₅ alkoxy, C₁₋₅ haloalkyl and C₁₋₅ haloalkoxy.

In some embodiments, in the compound shown in formula IIA, R₂ is a phenyl, and at least one hydrogen atom on the phenyl is substituted by hydroxyl.

In some embodiments, the compound is selected from the group consisting of:

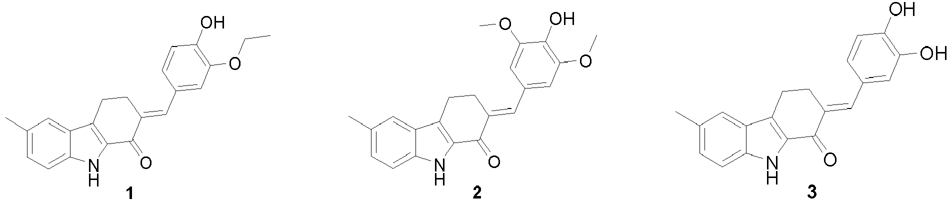

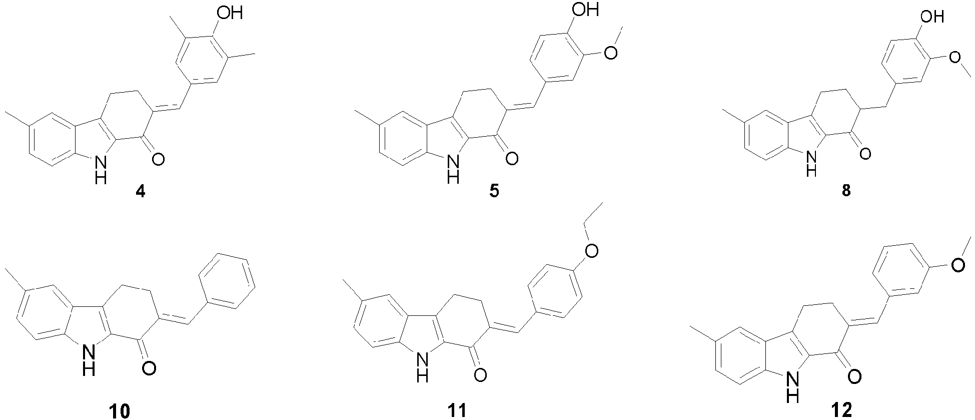

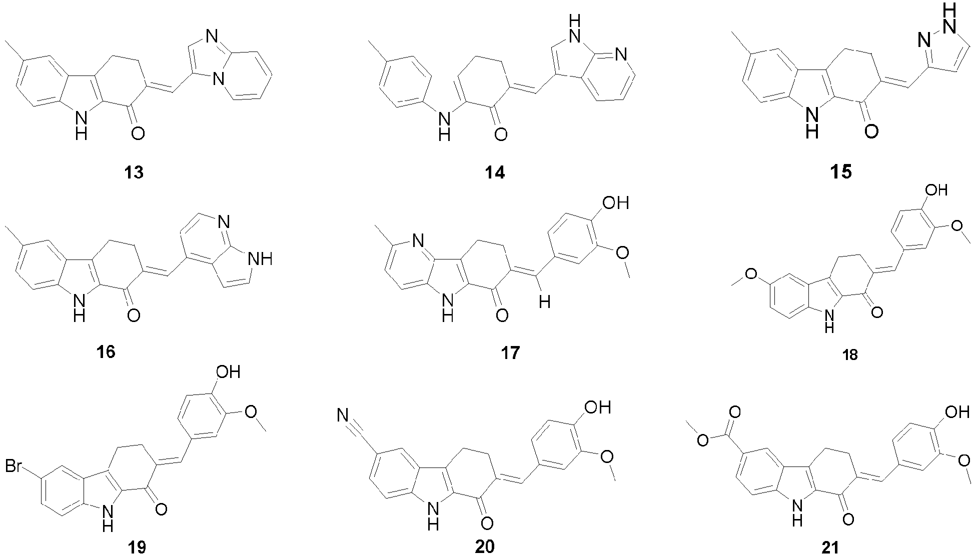

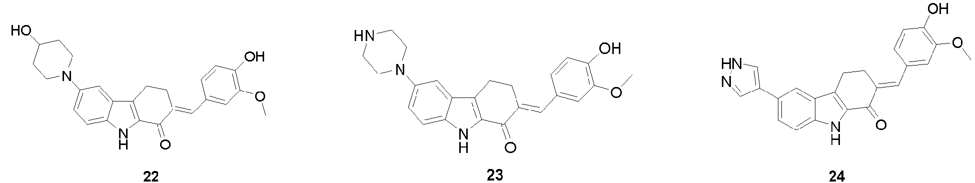

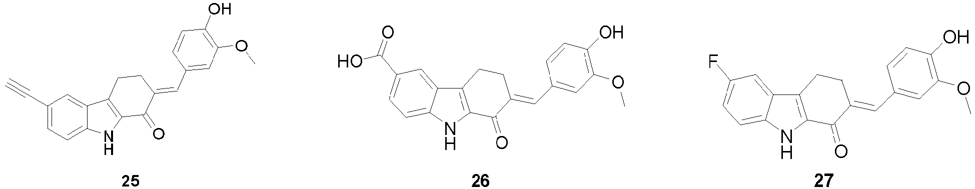

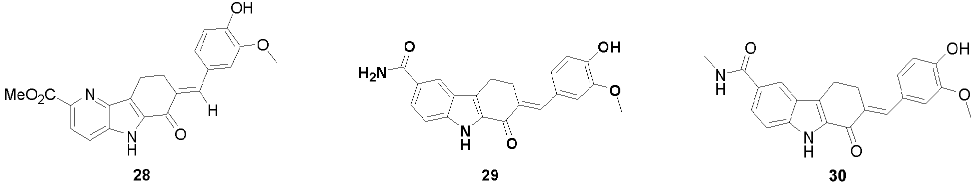

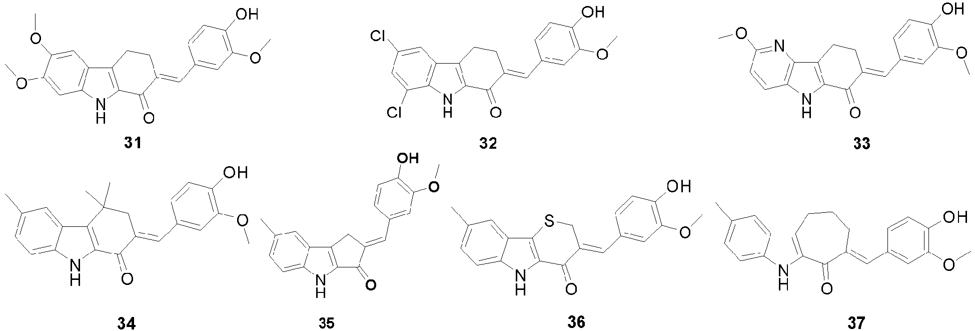

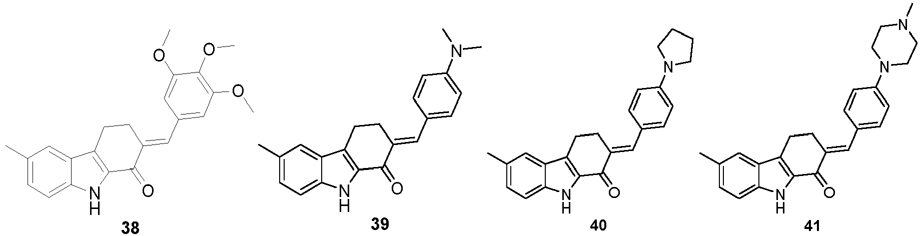

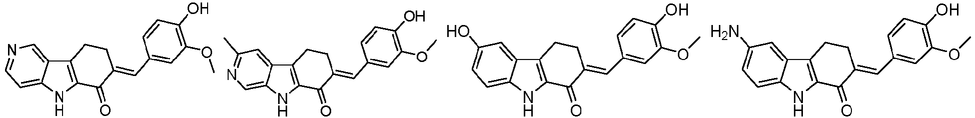

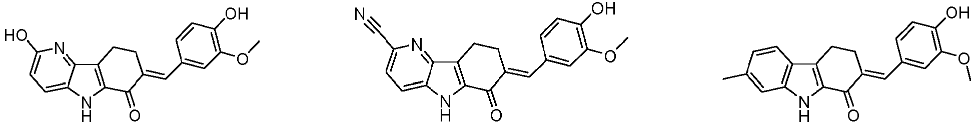

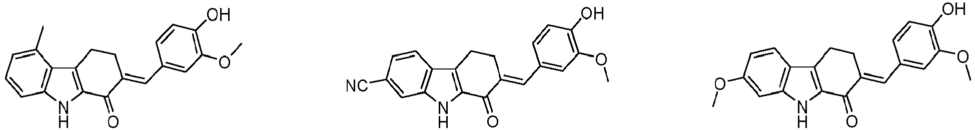

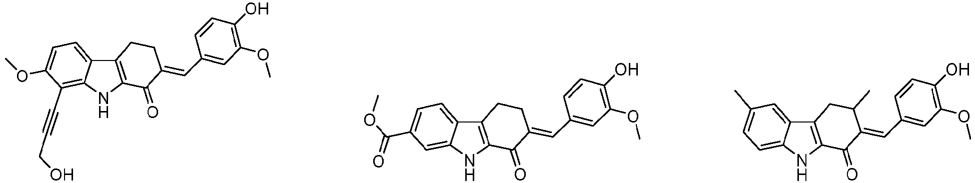

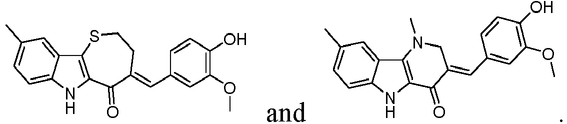

The second aspect of the present invention provided is a pharmaceutical composition, comprising (1) a compound according to the first aspect of the present invention, an optical isomer or a pharmaceutically acceptable salt thereof; and optional (2) pharmaceutically acceptable carriers, excipients or other active drugs.

In some embodiments, the pharmaceutical composition further comprises a second therapeutic component, and the second therapeutic component is a DNA methyltransferase inhibitor; preferably, the DNA methyltransferase inhibitor is SGI-1027.

The third aspect of the present invention provided is a use of a compound according to the first aspect of the present invention, an optical isomer or a pharmaceutically acceptable salt thereof, or a pharmaceutical composition according to the second aspect of the present invention in the preparation of a drug for treating and/or preventing cancers.

In some embodiments, the cancers are selected from the group consisting of liver cancer, lung cancer, breast cancer, pancreatic cancer, gastric cancer, cervical cancer, ovarian cancer, head and neck cancer.

In some embodiments, the treating and/or preventing cancers comprises: administrating the compound as shown in formula I according to any one of claims 1-9, an optical isomer or a pharmaceutically acceptable salt thereof, or the pharmaceutical composition, according to claim 7 in combination with a DNA methyltransferase inhibitor.

In some embodiments, the DNA methyltransferase inhibitor is SGI-1027.

It should be understood that within the scope of the present invention, the above-mentioned technical features of the present invention and the specific technical features described below (as embodiments) can be combined with each other to form new or preferred technical solutions. Due to space limitations, they will not be repeated herein.

### BRIEF DESCRIPTION OF THE DRAWINGS

Figure 1 is a schematic diagram of the synthesis method of some compounds provided in the present invention.
Figure 2 shows the inhibitory effect of some compounds on the OCT4 protein in HeLa cells.
Figure 3 shows the inhibitory effect of compounds KZT-A5 and AH057 on the transcription of the target gene of OCT4, NANOG, in HeLa cells.
Figure 4 shows the inhibitory effects of compounds KZT-A5 and AH057 on the JAK1/2-STAT3 signaling pathway in HeLa cells.
Figure 5 shows the inhibitory effect of compounds KZT-A5 and AH057 on the generation of tumor microspheres by tumor cells.
Figure 6 shows the effects of compounds KZT-A5 and AH057 on the viability of human tumor cell lines from different tissue sources. Wherein, Figure 6A shows the effect of AH057 on the viability of human tumor cell lines from different tissue sources, and Figure 6B shows the effect of KZT-A5 on the viability of human tumor cell lines from different tissue sources.
Figure 7 shows the in vitro proliferation inhibitory effect of compounds KZT-A5 single drug, SGI-1027 single drug, and KZT-A5+SGI-1027 synergistic therapy on HeLa cells. Figures 7A, 7B and 7C are photos of three technical repetitions.
Figure 8 shows the average statistical results of the inhibition effect on in vitro proliferation clone numbers of compounds KZT-A5 single drug, SGI-1027 single drug, and KZT-A5+SGI-1027 synergistic therapy on HeLa cells.

### DETAILED DESCRIPTION OF THE INVENTION

### Definitions

As used herein, the term "alkyl" includes straight or branched alkyl groups. For example, C₁₋₆ alkyl refers to a straight or branched alkyl having 1-6 carbon atoms, such as methyl, ethyl, n-propyl, isopropyl, cyclopropyl, n-butyl, isobutyl, tert-butyl, cyclobutyl, n-amyl, isopentyl, neopentyl, and the like.

As used herein, the term "alkenyl" includes straight or branched alkenyl groups. For example, C₂₋₆ alkenyl refers to a straight or branched alkenyl having2-6 carbon atoms, such as vinyl, allyl, 1-propenyl, isopropenyl, 1-butenyl, 2-butenyl, and the like.

As used herein, the term "alkynyl" includes straight or branched alkynyl groups. For example, C₂₋₆ alkyne refers to a straight or branched alkynyl having 2-6 carbon atoms, such as ethynyl, propenyl, butenyl, and the like.

As used herein, the term "cycloalkyl" refers to a cyclic saturated aliphatic hydrocarbyl having a specific number of carbon atoms. For example, C₃-C₁₀ alkenyl refers to a cyclic saturated aliphatic hydrocarbyl having3-10 carbon atoms. It can be a monocycle, such as cyclopropyl, cyclobutyl, cyclopentyl, cyclohexyl, and the like. "Cycloalkyl" can also be in the form of a bicyclic ring, such as a bridged ring or a spiro ring.

As used herein, the term "alkylamino" refers to the an amine group substituted byalkyl groups. For example, "C₁₋₆ alkylamino" refers to an amino substituted by a C₁₋₆ alkyl, which can be mono- or di-substituted; for example, methylamino, ethylamino, propylamino, iso-propylamino, butylamino, iso-butylamino, tert-butylamino, dimethylamino, diethylamino, dipropylamino, dibutylamino, di-iso-butylamino, di-tert-butylamino, and the like.

As used herein, the term "alkoxy" refers to a group having an alkyl-oxy structure. For example, "C₁₋₈ alkoxy" refers to a straight or branched alkoxy having 1-8 carbon atoms, including methoxy, ethoxy, propoxy, iso-propoxy, butoxy, iso-butyloxy, tert-butoxy, sec-butoxy, pentoxy, neopentoxy, and the like.

As used herein, the term "haloalkyl" refers to an alkyl in which one or more hydrogen atoms are substituted by halogens, wherein the definition of alkyl is as described above.

As used herein, the term "haloalkoxy" refers to an alkoxy in which one or more hydrogen atoms are substituted by halogens, wherein the definition of alkoxy is as described above.

As used herein, the term "carbonyl" refers to a group having a specific number of carbon atoms, and an alkyl-C(=O)- or alkyl-C(=O)-alkyl- structure.

As used herein, the term "heterocyclyl "heterocyclylalkyl" refers to a saturated or partially saturated cyclic group having a specific number of ring atoms (such as 3-10 ring atoms), wherein1-3 atoms are heteroatoms selected from N, S, and O. It can be a monocyclic, a bicyclic or a polycyclic form, such as bridged ring or spiro ring form. Specific examples can include oxetanyl, azetidinyl, tetrahydro-2H-pyranoyl, piperidyl, tetrahydrofuryl, morpholinyl, and pyrrolidinyl.

As used herein, the terms "aryl", "aromatic ring" or "aromatic-ring" refer to a aromatic cyclic group. For example, the term "C₆-C₁₀ aryl" refers to a aromatic group with 6-10 carbon atoms, such as phenyl or naphthyl. "Aryl" can also be a fused aryl.

As used herein, the term "heteroaryl" or "heteroaromatic ring" refers to an aromatic cyclic group in which 1-3 atoms are heteroatoms selected from the group consisting of N, S, and O; for example, the term "5-12 membered heteroaryl" refers to a heteroaryl having 5-12 atoms. It can be in the form of a mono- or a fused- ring. Specific examples can befuranyl, thienyl, pyrrolyl, thiazolyl, thiadiazolyl, azolyl, diazolyl, imidazolyl, pyrazolyl, pyridinyl, pyrimidinyl, 1,4-dioxacyclohexadienyl, 2H-1,2-azinyl, 4H-1,3-azinyl, 6H-1,2-azinyl, 4H-1,3-azinyl, 6H-1,3-azinyl, 4H-1,4-azinyl, pyridazinyl, pyrazinyl, 1,2,3-triazinyl,1,2,4-triazinyl, 1,3,5-triazinyl, 1,2,4,5-tetrazinyl, oxacycloheptatrienyl, thiacycloheptatrienyl, azacycloheptatrienyl, 1,3-diazacycloheptatrienyl, azacyclooctatetraenyl, benzofuranyl, benzoisofuranyl, benzothienyl, indolyl, benzozolyl, benzimidazolyl, indazolyl, benzotriazolyl, quinolyl, isoquinolinyl, pyridinopyrazolyl, pyridinopyrrolyl, pyrimidinopyrazolyl, pyrimidinopyrrolyl, pyridazinopyrazolyl, pyridazinopyrrolyl, acridinyl, phenanthridinyl, benzopyridazinyl, phthalazinyl, quinazolinyl, quinoxalinyl, phenazinyl, pteridinyl, purinyl, naphthyridinyl, and the like.

As used herein, "halogen" or "halogen atom" refers to F, Cl, Br and I. Preferably, the halogen or halogen atoms are selected from F, Cl, and Br. "Halogenated" refers to substituted by atoms selected from F, Cl, Br, and I.

Unless otherwise specified, the structural formulas described in the present invention are intended to include all isomeric forms (such as enantiomers, diastereomers, and geometric isomers (or conformational isomers): such as R and S configurations with asymmetric centers, (Z), (E) isomers with double bonds, etc. Therefore, mixtures of individual stereochemical isomers or their enantiomers, diastereomers, or geometric isomers (or conformational isomers) of the compounds of the present invention all fall within the scope of the present invention.

As used herein, the term "tautomer" refers to structural isomers having different energies that can exceed lower energy barriers and thus transform into each other. For example, proton tautomer (i.e. proton transfer) includes interconversion through proton transfer, such as 1H-indole and 2H-indole. The covalent tautomer include interconversion through the recombination of some bonding electrons.

As used herein, the term "solvate" refers to a complex formed by coordinating a compound of the invention with a solvent molecule in specific proportion.

As used herein, the term "hydrate" refers to a complex formed by coordinating a compound of the invention with water.

After extensive and intensive research, the inventor unexpectedly discovered a class of tetrahydrocarbazole compound and conducted a series of biological activity tests on it. It was found that it can jointly inhibit OCT4 at the transcriptional and functional levels by targeting OCT4 and JAK to promote CSC differentiation, and on the other hand, it can inhibit differentiated tumor cells by inhibiting the JAK/STAT pathway. Based on this, the present invention has been completed.

### The main advantages of the present invention are:

1. The tetrahydrocarbazole compounds of the present invention can jointly inhibit OCT4 at the gene transcription and functional level by targeting OCT4 and JAK to promote CSC differentiation, and inhibit differentiated tumor cells by inhibiting the JAK/STAT pathway, thereby specifically targeting the clearance of CSC and differentiated tumor cells, overcoming resistance to existing anti-tumor drugs, and efficiently inhibiting tumor proliferation, invasion and metastasis.
2. The tetrahydrocarbazole compounds of the present invention have good safety, low toxic side effects, and the prepared drugs thereof can be taken orally.
3. The combination of the tetrahydrocarbazole compounds of the present invention with other targeted drugs or chemotherapy drugs can achieve the goal of synergistic interaction, and has the potential to significantly increase the survival time of cancer patients and even cure certain types of cancer.
4. The tetrahydrocarbazole compounds of the present invention are first-class OCT4 inhibitors and the first inhibitors dual-targeting OCT4 and JAK1/2.

The present invention will be further illustrated below in conjunction with examples. Those skilled in the art will understand that the following examples are only intended to illustrate the present invention and should not be regarded as limiting the scope of the present invention. The specific technology or conditions with no specific conditions described in the examples are performed under the technology or conditions described in the literature in this field, or according to the product manual. The reagents or instruments used with no specific manufacturer are conventional products that can be commercially available.

### Example

### Example 1: Synthesis of compound 1

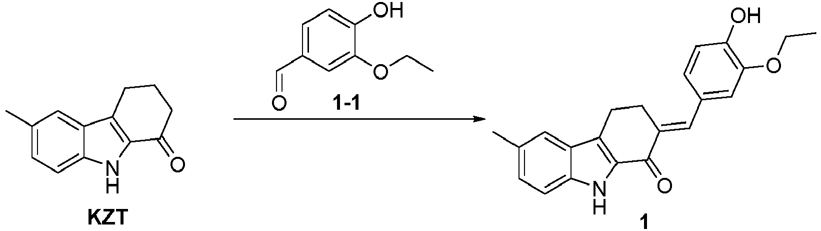

**KZT** (200 mg, 1.0 mmol), **1-1** (1.5 mmol), concentrated hydrochloric acid (10 mL) and methanol (5 mL) were added to a 25 mL single neck bottle in sequence, and they were refluxed for 10 hours until **KZT** disappears. The reaction solution was cooled down to room temperature and poured into crushed ice, the solid was precipitated, filtered, and the filter cake was washed with pure water. The dried crude product was purified by silica gel column chromatography (petroleum ether: ethyl acetate: methanol=100:10:1) to obtain a yellow green solid compound 1, yield 53.6%, m.p.=191-193 °C. ¹H NMR (400 MHz, DMSO-d₆) δ 11.60 (s, 1H), 9.39 (s, 1H), 7.57 (s, 1H), 7.46 (*s,* 1H), 7.33 (d, *J*=8.4 Hz, 1H), 7.17 (dd, *J*=8.5, 1.4 Hz, 1H), 7.11 (d, J=1.8 Hz, 1H), 7.03 (dd, *J*=8.4, 1.7 Hz, 1H), 6.89 (d, *J*=8.2 Hz, 1H), 4.09 (q, *J*=7.0 Hz, 2H), 3.23 (t, *J*=5.7 Hz, 2H), 3.01 (t, *J*=6.3 Hz, 2H), 2.40 (s, *3H),* 1.37 (t, *J*=7.0 Hz, 3H); 13C NMR (101 MHz, DMSO-d6) δ 180.24, 148.08, 147.05, 137.59, *135.11,* 134.52, 132.77, 129.04, 128.87, 127.40, 126.69, 125.86, 123.91, 120.80, 116.05, 115.95, 113.03, 64.43, 27.72, 21.55, 20.61, 15.22 HRMS (ESI) calcd for C₂₂H₂₀NO₃⁻ (M-H)⁻346.1449, found 346.1455.

### Example 2: Synthesis of compound 2

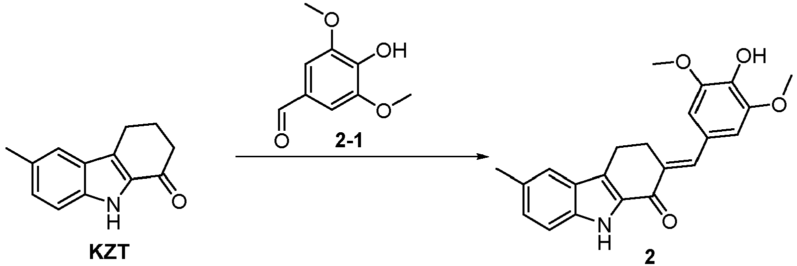

**KZT** (200 mg, 1.0 mmol), **2-1** (1.5 mmol), concentrated hydrochloric acid (10 mL) and methanol (5 mL) were added to a 25 mL single neck bottle in sequence, and they were refluxed for 10 hours until **KZT** disappears. The reaction solution was cooled down to room temperature and poured into crushed ice. The solid was precipitated, filtered, and the filter cake was washed with pure water. The dried crude product was purified by silica gel column chromatography (petroleum ether: ethyl acetate: methanol=100:10:1) to obtain a brown gel **2,** yield 43.7%.¹H NMR (400 MHz, CDCl₃) δ 9.53 (s, 1H), 7.76 (s, 1H), 7.43 (s, 1H), 7.36(d, *J*=*8.4* Hz, 1H), 7.21 (d, J=8.4 Hz, 1H), 6.72 (s, 2H), 3.93 (s, 6H), 3.29 (t, *J*=5.9 Hz, 2H), 3.06 (t, *J*=6.2 Hz, 2H), 2.45 (s, 3H) ¹³C NMR (101 MHz, CDCl₃) δ 176.72, 145.48, 144.40, 135.13, 132.61, 132.08, 130.15, 126.38, 126.30, 124.91, 124.10, 123.01, 121.24, 119.34, 112.50, 112.26,110.14, 55.66, 27.15, 21.00, 20.06 HRMS (ESI) calcd for C₂₂H₂₀NO₄⁻ (M-H) ⁻362.1398, found 362.1405..

### Example 3: Synthesis of compound 3

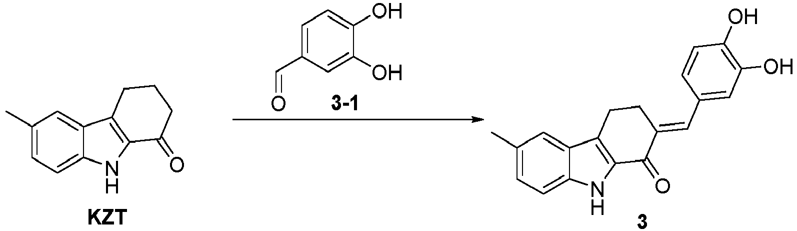

**KZT** (200 mg, 1.0 mmol), **3-1** (1.5 mmol), concentrated hydrochloric acid (10 mL) and methanol (5 mL) were added to a 25 mL single neck bottle in sequence, and they were refluxed for 10 hours until **KZT** disappears. The reaction solution was cooled down to room temperature and pouredinto crushed ice, the solid was precipitated, filtered, and the filter cake was washed with pure water. The dried crude product was purified by silica gel column chromatography (petroleum ether: ethyl acetate: methanol=100:10:1) to obtain a brown oil **3,** yield 43.5%.¹H NMR (400 MHz, DMSO-d₆) δ 11.58 (s, 1H), 7.47 (d, J=6.8 Hz, 2H), 7.32 (d, *J*=8.4 Hz, 1H), 7.16 (d, J=8.6 Hz, 1H), 6.99 (s, 1H), 6.91-6.77 (m, 2H), 3.20 (d, J=6.4 Hz, 2H), *3.00* (t, J=6.3 Hz, 2H), 2.39 (s, 3H) 13C NMR (101 MHz, DMSO-d6) δ 172.87, *145.69,* 137.55, 135.12, 134.08, 132.84, 128.91, 128.74, 126.46, 126.46, 125.86, 122.91, 120.80, 117.64, 117.48, 116.20, 113.00, 27.73, 21.57, 20.62 HRMS (ESI) calcd for C₂₀H₁₆NO₃⁻(M-H)⁻318.1136, found 3118.1140.

### Example 4: Synthesis of compound 4

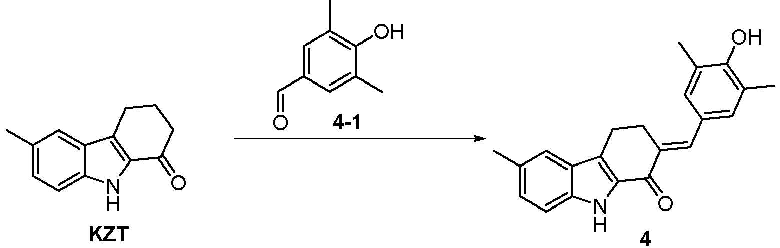

**KZT** (200 mg, 1.0 mmol), **4-1** (1.5 mmol), concentrated hydrochloric acid (10 mL) and methanol (5 mL) were added to a 25 mL single neck bottle in sequence, and they were refluxed for 10 hours until **KZT** disappears. The reaction solution was cooled down to room temperature and poured into crushed ice, the solid was precipitate, filtered, and the filter cake was washed with pure water. The dried crude product was purified by silica gel column chromatography (petroleum ether: ethyl acetate: methanol=100:10:1) to obtain a brown solid **4,** yield 52.7%, m.p.=195-197 °C. ¹HNMR (400 MHz, DMSO-d₆) δ 11.56 (s, 1H), 8.67 (s, 1H), 7.49 (s, 1H), 7.43 (s, 1H), 7.31 (d, J=8.5 Hz, 1H), 7.17-7.12 (m, 3H), 3.20 (t, J=5.6 Hz, 2H), 2.97 (t, J=6.3 Hz, 2H), 2.38 (s, 3H), 2.21 (s, 6H).¹³C NMR (101 MHz, DMSO-d₆) δ 180.35, 154.51, 137.59, 135.05, 134.32, 132.82, 130.91, 129.02, 128.83, 127.07, 126.61, 125.88, 124.80, 120.80, 113.04, 27.76, 21.56, 20.64, 17.15 HRMS (ESI) calcd for C₂₂H₂₀NO₂'(M-H)⁻330.1500, found 330.1505.

### Example 5: Synthesis of compound 5

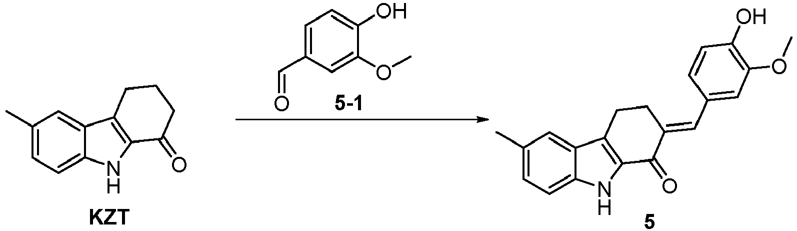

**KZT** (200 mg, 1.0 mmol), **5-1** (1.5 mmol), concentrated hydrochloric acid (10 mL) and methanol (5 mL) were added to a 25 mL single neck bottle in sequence, and they were refluxed for 10 hours until **KZT** disappears. The reaction solution was cooled down to room temperature and poured into crushed ice, the solid was precipitated, filtered, and the filter cake was washed with pure water. The dried crude product was purified by silica gel column chromatography (petroleum ether: ethyl acetate: methanol=100:10:1) to obtain a yellow solid **5,** yield 45.7%, m.p.=169-171 °C. ¹H NMR (400 MHz, DMSO-d₆) δ 11.60 (s, 1H), 9.46 (s, 1H), 7.57 (s, 1H), 7.46∼7.42 (m, 1H),7.31 (d,J=8.4 Hz, 1H), 7.15 (dd, J=8.5, 1.3 Hz, 1H), 7.11 (d, J=1.8 Hz, 1H), 7.02 (dd, *J*=8.3, 1.6 Hz, 1H), 6.87 (d, J=8.1 Hz, 1H), 3.82 (s, 3H), 3.27∼3.16 (m, 2H), 2.99 (t, *J*=6.3 Hz, 2 H), 2.38 (s, 3H).¹³C NMR (101 MHz, DMSO-d₆) δ 180.18, 147.94, 147.86, 137.59, 135.07, 134.54, 132.81, 128.94, 128.79, 127.41, 126.56, 125.87, 123.80, 120.80, 115.96, 114.82, 113.00, *55.68,* 27.25, 21.10, 20.16 HRMS (ESI) calcd for C₂₁H₁₈NO₃⁻ (M-H) ⁻332.1292, found 332.1296.

### Example 6: Synthesis of compound 8

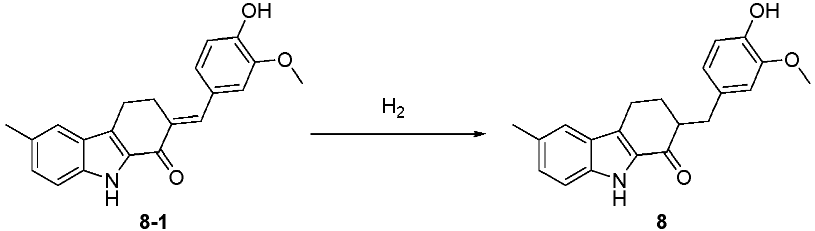

Palladium hydroxide on carbon (25 mg, 0.180 mmol) was added to a solution of compound **8-1** (300 mg, 0.900 mmol) in ethyl acetate (1 mL) at room temperature under a hydrogen atmosphere. The reaction was stirred overnight at room temperature, and then filtered, the filter cake was washed with ethyl acetate (2 × 10 mL). After the filtrate was concentrated, it was separated by reverse phase preparation column (mobile phase A: water (10 mmol/L ammonium bicarbonate), mobile phase B: acetonitrile) to obtain a white solid compound **8** (54.8 mg, 17.85%). ESI/MS (m/z): 366.1 [M+H]⁺. ¹HNMR (400 MHz, DMSO-d₆): δ 11.46 (s, 1H), 8.72 (s, 1H), 7.40 (s, 1H), 7.29 (d, J=8.4 Hz, 1H), 7.16-7.08 (m, 1H), 6.82 (d, *J*=1.6 Hz, 1H), 6.70 (d, J=7.9 Hz, 1H), 6.63 (dd, *J*=8.0, 1.7 Hz, 1H), 3.75 (s, 3H), 3.19 (dd, *J*=13.7, 3.9 Hz, 1H), 2.99 (dt, *J*=16.4, 4.4 Hz, 1H), 2.89 5-2.71 *(m,* 2H), 2.59-2.52 (m, 1H), 2.37 (s, 3H), 2.15-2.01 (m, 1H), 1.78 (m, 1H).

### Example 7: Synthesis of compound 10

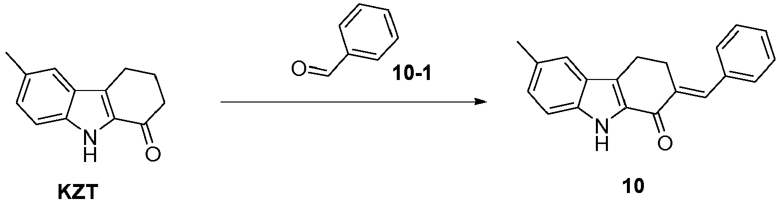

Concentrated sulfuric acid (0.2 mL, 3.752 mmol) was added to a mixture of compound **KZT** (150 mg, 0.753 mmol) and **10-1** (95 mg, 0.904 mmol) in methanol (2 mL) at room temperature. The reaction solution was stirred at 60 °C overnight, and then adjusted to pH 8 with sodium hydroxide. After concentration, the crude product was separated by reverse phase preparation column (mobile phase A: water (10 mmol/L ammonium bicarbonate), mobile phase B: acetonitrile) to obtain a yellow solid compound **10** (49.3 mg, 22.79%). ESI/MS (m/z): 288.2 [M+H]⁺. ¹H NMR (400 MHz, DMSO-d₆): δ 11.66 (s, 1H), 7.62 (s, 1H), 7.52 (d, J=7.3 Hz, 2H), 7.49 (s, 1H), 7.46 (d, *J*=7.3 Hz, 2H), 7.39 (t, *J*=7.2 Hz, 1H), 7.33 (d, *J*=8.5 Hz, 1H), 7.17 (dd, *J*=8.5, 1.3 Hz, 1H), 3.23-3.14 (m, 2H), 3.00 (t, *J=6.2* Hz, 2H), 2.38 (s, 3H).

### Example 8: Synthesis of compound 11

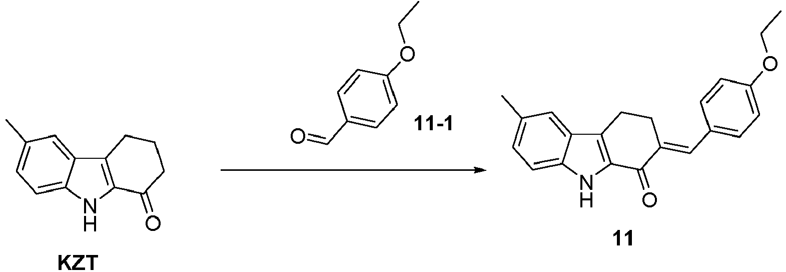

Concentrated sulfuric acid (0.2 mL, 3.752 mmol) was added to a mixture of compound **KZT** (150 mg, 0.753 mmol) and **11-1** (135 mg, 0.904 mmol) in methanol (2 mL) at room temperature. The reaction solution was stirred overnight at 60 °C,and then adjusted to pH 8 with sodium hydroxide. After concentration, the crude product was separated by reverse phase preparation column (mobile phase A: water (10 mmol/L ammonium bicarbonate), mobile phase B: acetonitrile) to obtain a yellow solid compound **11** (8.9 mg, 3.57%). ESI/MS (m/z): 332.1 [M+H]⁺. ESI/MS (m/z): 332.1 [M+H]⁺. ¹HNMR (400 MHz, DMSO-d6) δ 11.61 (s, 1H), 7.57 (d, *J*=2.1 Hz, 1H), 7.52-7.46 (m, 2H), 7.45 (s, 1H), 7.32 (d, J=8.4 Hz, 1H), 7.16 (dd, J=8.5, 1.7 Hz, 1H), 7.05-6.95 (m, 2H), 4.08 (q, *J*=*7.0* Hz, 2H), 3.20 (td, J=6.4, 1.8 Hz, 2H), 3.00 (t, *J*=6.3 Hz, 2H), 2.38 (s, 3H), 1.35 (t,J=7.0 Hz, 3H).

### Example 9: Synthesis of compound 12

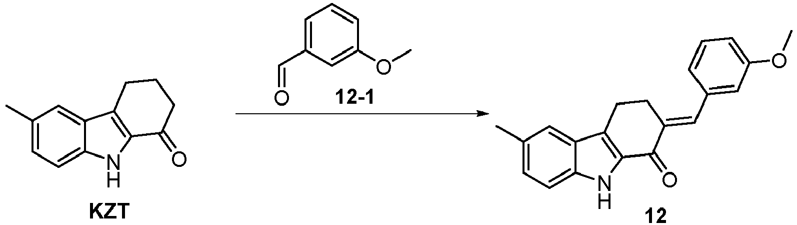

**KZT** (200 mg, 1.0 mmol), **12-1** (1.5 mmol), concentrated hydrochloric acid (10 mL) and methanol (5 mL) were added to a 25 mL single neck bottle in sequence, and they were refluxed for 10 hours until KZT disappears. The reaction solution was cooled down to room temperature and poured into crushed ice, the solid was precipitated, filtered, and the filter cake was washed with pure water. The dried crude product was purified by silica gel column chromatography (petroleum ether: ethyl acetate: methanol=100:10:1) to obtain a yellow green solid compound **12,** yield 46.7%, m.p.=200-202 °C. ¹HNMR (400 MHz, DMSO-d₆) δ 11.66 (s, 1H), 7.60 (s, 1H), 7.47 (s, 1H), 7.40 (t, *J*=7.9 Hz, 1H), 7.34 (d, *J*=8.4 Hz, 1H), 7.21-7.15 (m, 1H), 7.11 (d, *J*=7.8 Hz, 1H), 7.08 (s, 1H), 6.98 (dd, J=8.2, 2.4 Hz, 1H), 3.82 (s, 3H), 3.24-3.16 (m, 2H), 3.02 (t, *J*=6.3 Hz, 2H), 2.40 (s) 3H) 13C NMR (101 MHz, DMSO-d₆) δ 180.08, 148.14, 138.09, 135.47, 134.84, 132.61, 128.64, 128.99, 127.81, 126.86, 124.87, 122.60, 120.60, 118.17, 115.26, 114.42, 113.02, 55.66, 27.21, 21.08, 20.06 HRMS (ESI) calcd for C₂₁H₁₈NO₂⁻(M-H)⁻316.1343, found 316.1346.

### Example 10: Synthesis of compound 13

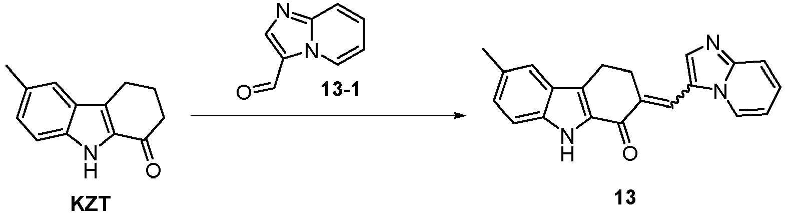

Potassium hydroxide (211 mg, 3.765 mmol) was added to a solution of compound **KZT** (150 mg, 0.753 mmol) and **13-1** (132 mg, 0.904 mmol) in methanol (3 mL) at room temperature. The reaction solution was stirred overnight at 65 °C, and then adjusted to pH 8 with 2M hydrochloric acid. After concentration, the crude product was separated by reverse phase preparation column (mobile phase A: water (10 mmol/L+0.1% ammonia water), mobile phase B: acetonitrile) to obtain a yellow solid compound **13** (5.0 mg, 2.03%). ESI/MS (m/z): 328.1 [M+H]⁺. ¹HNMR (400 MHz, DMSO-d₆): δ 11.65 (s, 1H), 11.61 (s, 1H), 8.84 (s, 1H), 8.83 (d, *J*=7.2 Hz, 1H), 8.74 (d, J=6.9 Hz, 1H), 8.11 (s, 1H), 7.89 (s, 1H), 7.71 (d, *J*=9.1 Hz, 1H), 7.66 (d, *J*=9.0 Hz, 1H), 7.48 (s, 1H), 7.47-7.45 (m, 1H), 7.45-7.40 (m, 1H), 7.40-7.36 (m, 1H), 7.45-7.40 (m, 1H) 34 (s, 1H), 7.32 (s, 1H), 7.30 (s, 1H), 7.19-7.13 (m, 2H), 7.12-7.06 (m, 2H), 3.31 (s, 2H), 3.18 (t, J=5.6 Hz, 2H), 3.10 (d, J=6.4 Hz, 2H), 3.07 (d, J=6.1 Hz, 2H), 2.40 (s, 3H), 2.39 (s, 3H).

### Example 11: Synthesis of compound 14

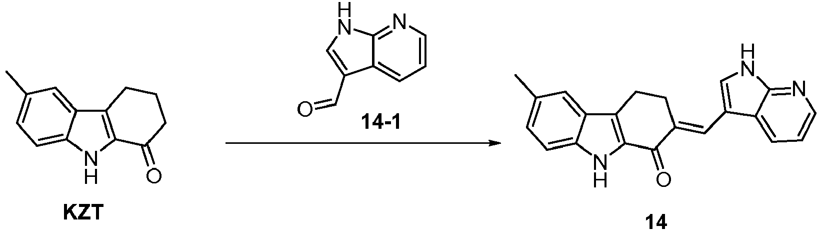

Concentrated sulfuric acid (0.4 mL, 7.504 mmol) was added to a solution of compound **KZT** (409 mg, 2.053 mmol) and **14-1** (300 mg, 2.053 mmol) in methanol (4 mL) at room temperature. After stirred overnight at 65 °C, the reaction solution was adjusted to pH 8 with a saturated sodium bicarbonate aqueous solution. After concentration, the crude product was separated by reverse phase preparation column (mobile phase A: water (10 mmol/L), mobile phase B: acetonitrile) to obtain a brown solid compound **14** (6.3 mg, 0.94%). ESI/MS (m/z): 328.0 [M+H]⁺.

### Example 12: Synthesis of compound 15

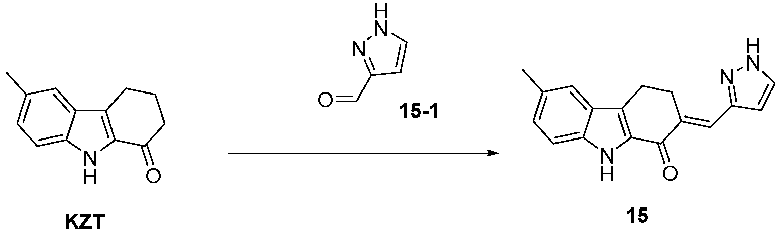

Potassium hydroxide (437 mg, 7.805 mmol) was added to a solution of compound **KZT** (311 mg, 1.561 mmol) and **15-1** (150 mg, 1.561 mmol) in methanol (4 mL) at room temperature. The reaction solution was stirred overnight at 65 °C, and then adjusted to pH 8 with 2M hydrochloric acid. After concentration, the reaction solution was separated by reverse phase preparation column (mobile phase A: water (10 mmol/L ammonium bicarbonate+0.1% ammonia water), mobile phase B: acetonitrile) to obtain a yellow solid compound **15** (32.3 mg, 7.41%).ESI/MS (m/z): 278.1 [M-H] ⁻.¹HNMR (400 MHz, DMSO-d₆): δ 13.21 (s, 1H), 11.61 (s, 1H), 7.82 (s, 1H), 7.52 (s, 1H), 7.47 (s, 1H), 7.32 (d, J=8.5 Hz, 1H), 7.17 (dd, J=8.5, 1.3 Hz, 1H), 6.66 (s, 1H), 3.53-3.37 (m, 2H), 3.04 (t, J=6.2 Hz, 2H), 2.39 (s, 3H).

### Example 13: Synthesis of compound 16

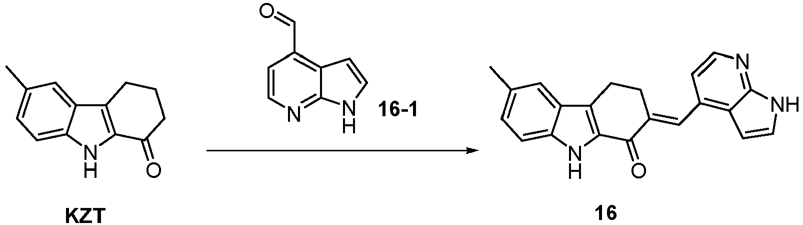

Potassium hydroxide (42.5 mg, 0.759 mmol) was added to a mixture of **KZT(37.0** mg, 0.253 mmol) and **16-1** (60.5 mg, 0.303 mmol) in 1,4-dioxane (2 mL) at room temperature. The reaction was heated to 100 °C for 3 hours. It was then quenched with water, and the obtained mixture was extracted with ethyl acetate (3 × 20 mL). The combined organic layer was washed with saturated salt water (5 mL), dried over anhydrous sodium sulfate, filtered and concentrated under reduced pressure. The crude product was separated and purified by silica gel column chromatography (methanol: dichloromethane=1:20) to obtain a yellow solid compound **16** (2.0 mg, 2.41%). ESI/MS (m/z) 327.9 [M+H]⁺.

### Example 14: Synthesis of compound 17

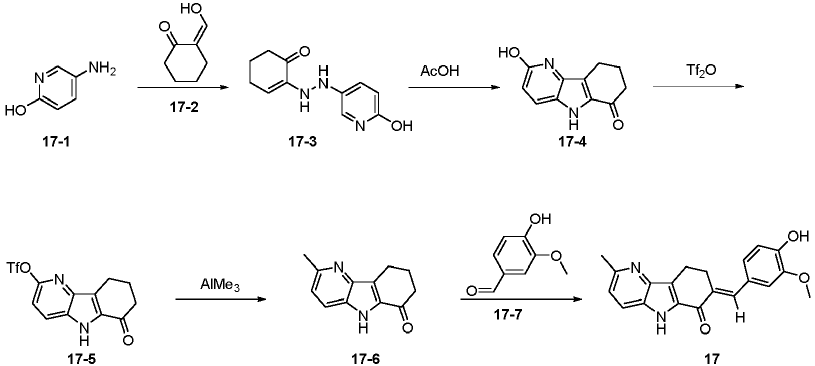

An aqueous solution (20 mL) of sodium nitrite (1.25 g, 18.162 mmol) was added slowly to an aqueous solution (83 mL) of compound**17-1** (1 g, 9.081 mmol) and concentrated hydrochloric acid (2.70 mL, 88.903 mmol) under zero temperature conditions. The reaction solution was stirred at 0 °C for 30 minutes, then a solution (80 mL, 1:1) of compound **17-2** (1.15 g, 9.081 mmol) and sodium acetate (1.64 g, 19.978 mmol) in methanol and water was added slowly to the reaction solution. The reaction solution was stirred at 0 °C for additional 0.5 hours, and then adjusted to pH 8 with a saturated sodium bicarbonate aqueous solution. After concentration, the crude product was subjected to silica gel column chromatography (dichloromethane: methanol=5:1) to obtain a yellow solid compound **17-3** (400 mg, 20.09%). ESI/MS (m/z): 220.1 [M+H]^{+.}

Acetic acid (12 mL, 209.359 mmol) and concentrated sulfuric acid (1.20 mL, 22.508 mmol) were added to a solution of compound **17-3** in methanol (3 mL) at room temperature. The reaction solution was refluxed and stirred at 65 °C for 2 hours, and then adjusted to pH 8 with a saturated sodium bicarbonate aqueous solution. After concentration, the crude product was separated by silica gel column chromatography (dichloromethane: methanol=5:1) to obtain a yellow solid compound **17-4** (300 mg, 43.42%). ESI/MS (m/z): 203.1 [M+H]⁺.

Pyridine (587 mg, 7.420 mmol) and trifluoromethanesulfonic anhydride (935 mg, 4.452 mmol) were added to a mixture of compound **17-4** (300 mg, 1.484 mmol) in dichloromethane (10 mL) at room temperature, and stirred for 2 hours. After concentration, the crude product was subjected to silica gel column chromatography (petroleum ether: ethyl acetate=1:1) to obtain a yellow solid compound **17-5** (210 mg, 42.35%). ESI/MS (m/z): 334.8 [M+H]⁺.

Under a nitrogen atmosphere conditions, trimethylaluminum (226 mg, 3.143 mmol) was added slowly to a mixture of compounds **17-5** (150 mg, 0.449 mmol) and Pd(PPh₃)₄ (52 mg, 0.045 mmol) in tetrahydrofuran (5 mL) at 0 °C. The reaction solution was refluxed at 80 °C for 1 hour. It was then concentrated, and subjected to silica gel column chromatography (petroleum ether: ethyl acetate=1:1) to obtain a yellow solid compound **17-6** (70 mg, 77.90%). ESI/MS (m/z): 200.9 [M+H]⁺.

Concentrated sulfuric acid (0.1 mL) was added to a mixture of compounds **17-6(60** mg, 0.300 mmol) and **17-7(50** mg, 0.330 mmol) in methanol (1 mL) at room temperature. The reaction solution was stirred and refluxed overnight at 65 °C, and then adjusted to pH 8 with a saturated sodium bicarbonate aqueous solution. After concentration, the crude product was separated by reverse phase preparation column to obtain a yellow solid compound **17** (43.8 mg, 42.54%).ESI/MS (m/z): 335.0 [M+H]⁺. ¹HNMR (400 MHz, DMSO-d₆): δ 11.86 (s, 1H), 9.54 (s, 1H), 7.72 (d, J=8.5 Hz, 1H), 7.63 (s, 1H), 7.20 (dd, J=8.4, 3.8 Hz, 1H), 7.15 (s, 1H), 7.06 (d, *J*=8.3 Hz, 1H), 6.90 (d, J=8.1 Hz, 1H), 3.84 (s, 3H), 3.26 (t, *J*=6.0 Hz, 2H), *3.07* (t, J=6.2 Hz, 2H), 2.57 (s, 3H).

### Example 15: Synthesis of compound 18

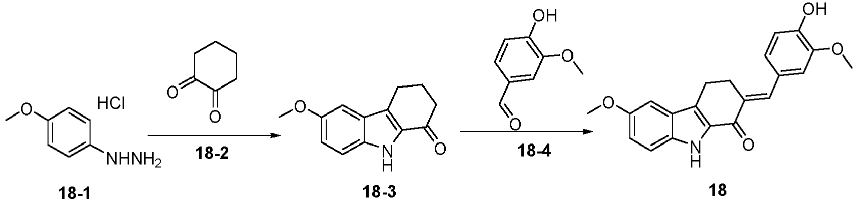

Concentrated hydrochloric acid (1 mL) was added to a solution of compounds **18-1** (1 g, 7.237 mmol) and **18-2** (0.97 g, 8.684 mmol) in 10 mL acetic acid at room temperature. After stirred at 65 °C for 2 hours, the reaction solution was directly concentrated and separated by silica gel column chromatography (petroleum ether: ethyl acetate=8:1) to obtain a light brown solid compound **18-3** (110 mg, 7.06%). ESI/MS (m/z): 216.2 [M+H]⁺. ¹H NMR (400 MHz, DMSO-d₆): δ 11.44 (s, 1H), 7.30 (d, J=8.8 Hz, 1H), 7.10 (s, 1H), 6.97 (d, J=8.8 Hz, 1H), 3.78 (s, 3H), 2.92 (s, 2H), 2.54 (s, 2H), 2.14 (s, 2H).

Concentrated sulfuric acid (0.3 mL, 5.628 mmol) was added to a solution of compounds **18-3(110** mg, 0.511 mmol) and **18-4** (93 mg, 0.613 mmol) in methanol (3 mL) at room temperature. The reaction solution was stirred overnight at 65 °C, and then adjusted to pH 8 with a saturated sodium bicarbonate aqueous solution, and extracted with ethyl acetate (3 × 20mL). The organic phase was washed with saturated saline water (30 mL), dried over anhydrous sodium sulfate, concentrated, and the crude product was separated by reverse phase preparation column (mobile phase A: water (10 mmol/L ammonium bicarbonate), mobile phase B: acetonitrile) to obtain a yellow solid compound **18** (5.9 mg, 3.30%).ESI/MS (m/z): 350.0 [M+H]⁺. ¹H NMR (400 MHz, DMSO-d₆): δ 11.58 (s, 1H), 9.44 (s, 1H), 7.56 (s, 1H), 7.32 (d, *J*=8.9 Hz, 1H), 7.11 (t, *J*=2.4 Hz, 2H), 7.03 (dd, J=8.3, 1.5 Hz, 1H), 6.98 (dd, *J*=9.0, 2.5 Hz, 1H), 6.86 (d, *J*=8.1 Hz, 1H), 3.82 (s, 3H), 3.79 (s, 3H), 3.22 (t, J=5.8 Hz, 2H), 3.00 (t, J=6.3 *Hz,* 2H) H).

### Example 16: Synthesis of compound 19

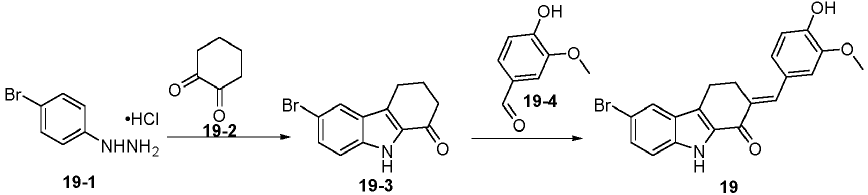

AcOH acetate (15 mL) and concentrated hydrochloric acid (3 mL) were added to a solution of compounds **19-1** (5 g, 22.371 mmol) and **19-2** (3.01 g, 26.845 mmol) in methanol (60 mL) at room temperature. The reaction solution was stirred overnight at 65 °C, and then adjusted to pH 8 with a saturated sodium bicarbonate aqueous solution. After concentration, it was separated by silica gel column chromatography (petroleum ether: ethyl acetate=5:1) to obtain a light yellow compound **19-3** (700 mg, 11.85%).ESI/MS (m/z): 263.8 [M+H]⁺.

Concentrated sulfuric acid (1.5 mL, 28.143 mmol) was added to a mixture of compounds **19-3** (300 mg, 1.136 mmol) and **19-4** (259 mg, 1.704 mmol) in MeOH (15 mL) at room temperature, and the reaction solution was stirred overnight at 80 °C. The reaction solution was then cooled down to room temperature, and adjusted to pH 8 with a saturated sodium bicarbonate aqueous solution, extracted with ethyl acetate and concentrated to obtain a crude product, it was then separated by reverse phase preparation column to obtain a yellow solid compound **19** (66.8 mg, 14.80%). ESI/MS (m/z): 398.0 [M+H]⁺.¹HNMR (400 MHz, DMSO-d₆): δ 11.94 (s, 1H), 9.48 (s, 1H), 7.93 (s, 1H), 7.59 (s, 1H), 7.43 (dd, J=8.8, 1.9 Hz, 1H), 7.38 (d, *J*=8.6 Hz, 1H), 7.12 (d, J=1.7 Hz, 1H), 7.04 (dd, J=8.2, 1.6 Hz, 1H), 6.87 (d, J=8.1 Hz, 1H), 3.82 (s, 3H), 3.28-3.19 (m, 2H), 3.02 (t, J=6.3 Hz, 2H)).

### Example 17: Synthesis of compound 20

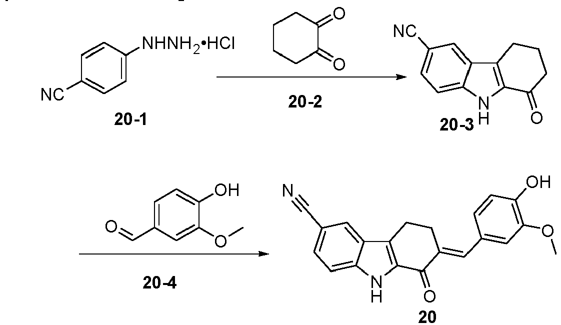

Concentrated hydrochloric acid (2.4 mL, 78.991 mmol) was added to solution of **20-1** (2.5 g, 15.020 mmol) and **20-2** (2.02 g, 18.024 mmol) in anacetic acid (11.8 mL) at room temperature. The reaction solution was refluxed at 120 °C for 1 hour. The reaction solution was then concentrated, and separated by silica gel column (petroleum ether: ethyl acetate=1:1) to obtain a yellow solid compound **20-3** (400 mg, 12.67%). ESI/MS (m/z): 211.1 [M+H]⁺.

Concentrated sulfuric acid (0.5 mL, 9.381 mmol) was added to a solution of **20-3** (200 mg, 0.951 mmol) and **20-4** (173.69 mg, 1.141 mmol) in methanol (5 mL) at room temperature. The reaction solution was stirred overnight at 65 °C, and then adjusted to pH 8 witha saturated sodium bicarbonate aqueous solution, extracted with ethyl acetate (3 × 20 mL), and the organic phase was washed with saturated salt water, dried over anhydrous sodium sulfate, filtered and concentrated. The crude product was prepared and separated by reverse phase column (mobile phase A: 0.05% ammonia water, mobile phase B: acetonitrile) to obtain a yellow solid compound **20** (15.6 mg, 4.65%). ESI/MS (m/z): 345.0 [M+H]⁺. ¹H NMR (400 MHz, DMSO-d₆): δ 12.28 (s, 1H), 8.34 (s, 1H), 7.64 (dd, J=8.6, 1.4 Hz, 1H), 7.61 (s, 1H), 7.56 (d, J=8.6 Hz, 1H), 7.15-7.10 (m, 1H), 7.09-6.99 (m, 1H), 6.87 (d, J=8.2 Hz, 1H), 3.82 (s, 3H), 3.28-3.25 (m, 2H), 3.07 (t, J=6.8 Hz, 2H).

### Example 18: Synthesis of compound 21

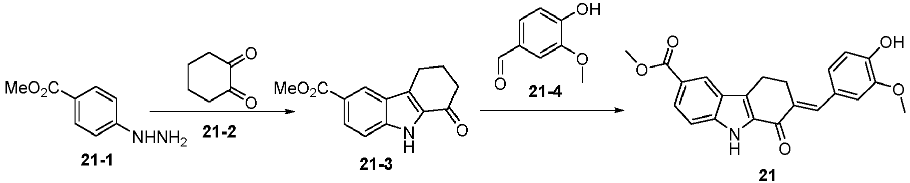

Concentrated sulfuric acid (1 mL, 18.762 mmol) was added to a solution of compounds **21-1** (920 mg, 4.540 mmol) and **21-2** (610 mg, 5.448 mmol) in acetic acid (10 mL) at room temperature. The reaction solution was stirred at 65 °C for 2 hours, and then adjusted to pH 8 with a saturated sodium bicarbonate aqueous solution, extracted with ethyl acetate (3 × 50 mL), dried over anhydrous sodium sulfate and concentrated, the crude product was separated by silica gel column chromatography (petroleum ether: ethyl acetate=2:1) to obtain a brown solid compound **21-3** (480 mg, 43.46%). ESI/MS (m/z): 244.2 [M+H]⁺.

Concentrated sulfuric acid (0.4 mL, 7.505 mmol) was added to a solution of compounds **21-3** (150 mg, 0.617 mmol) and **21-4** (112 mg, 0.740 mmol) in methanol (4 mL) at room temperature. The reaction solution was stirred overnight at 80 °C, and then adjusted to pH 8 with a saturated sodium bicarbonate aqueous solution. After concentration, the crude product was separated by reverse phase preparation column (mobile phase A: water (0.05% formic acid), mobile phase B: acetonitrile) to obtain a yellow solid compound **21** (20.04 mg, 8.35%). ESI/MS (m/z): 378.2 [M+H]⁺. ¹HNMR (400 MHz, DMSO-d₆): δ 12.16 (s, 1H), 9.50 (s, 1H), 8.4-8.31 (m, 1H), 7.92 (dd, J=8.7, 1.6 Hz, 1H), 7.60 (s, 1H), 7.50 (d, J=8.8 Hz, 1H), 7.13 (d, *J*=1.8 Hz, 1H), 7.05 (dd, J=8.2, 1.6 Hz, 1H), 6.87 (d, J=8.1 Hz, 1H), 3.86 (s, 3H), 3.83 (s, 3H), 3.26 (t, *J*=5.2 Hz, 2H), 3.10 (t, J=6.3 Hz, 2H).

### Example 19: Synthesis of compound 22

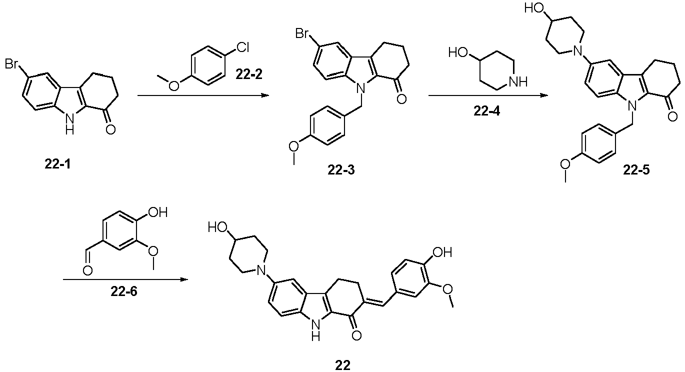

Under a nitrogen atmosphere, sodium hydride NaH (29.9 mg, 1.250 mmol) was added to a solution of compounds **22-1** (300 mg, 1.136 mmol) and **22-2** (162 mg, 1.136 mmol) in DMF (5 mL) at 0 °C. The reaction solution was stirred at room temperature for 4 hours. The reaction was then quenched with water, extracted with ethyl acetate (3 × 20 mL), washed with saturated salt water, dried over anhydrous sodium sulfate, concentrated, and then the crude product was separated by silica gel column chromatography (petroleum ether: ethyl acetate=5:1) to obtain a white solid compound **22-3** (300 mg, 68.73%). ESI/MS (m/z): 384.0 [M+H]⁺.

Under a nitrogen atmosphere, catalyst Pd PEPPSI-IHeptCl₃-chloropyridine (76 mg, 0.078 mmol) was added to a solution of compounds **22-3** (300 mg, 0.781 mmol), Cs₂CO₃ (763 mg, 2.343 mmol) and **22-4** (236.9 mg, 2.343 mmol) in 1,4-dioxane (8 mL) at room temperature. The reaction solution was stirred overnight at 100 °C under a nitrogen atmosphere, then cooled and concentrated, the crude product was then separated by silica gel column chromatography (dichloromethane: methanol=1:1) to obtain a light yellow solid compound **22-5** (150 mg, 47.50%). ESI/MS (m/z): 405.2 [M+H]⁺.

Concentrated sulfuric acid (0.5 mL, 9.381 mmol) was added to a solution of compounds **22-5** (150 mg, 0.371 mmol) and **22-6** (68 mg, 0.445 mmol) in methanol (5 mL) at room temperature. The reaction solution was stirred overnight at 65 °C, and then adjusted to pH 8 with a saturated sodium bicarbonate aqueous solution, extracted with ethyl acetate (3 × 20 mL), washed with saturated salt water (30 mL), dried over anhydrous sodium sulfate, concentrated, and then the crude product was separated by reverse phase preparation column (mobile phase A: water (0.05% formic acid), mobile phase B: acetonitrile) to obtain a yellow solid compound **22** (25.3 mg, 15.99%). ESI/MS (m/z): 419.1 [M+H]⁺. ¹HNMR (400 MHz, DMSO-d₆): δ 11.46 (s, 1H), 9.43 (s, 1H), 7.55 (s, 1H), 7.28 (d, *J*=9.0 Hz, 1H), 7.15 (dd, J=9.1, 2.2 Hz, 1H), 7.10 (d, *J*=1.5 Hz, 1H), 7.03 (s, 1H), 7.02-6.99 (m, 1H), 6.86 (d, J=8.1 Hz, 1H), 4.66 (d, J=3.8 Hz, 1H), 3.82 (s, 3H), 3.59 (dq, J=8.9, 4.4 Hz, 1H) 3.47-3.36 (m, 2H), 3.21 (t, J=5.6 Hz, 2H), 2.98 (t, *J*=6.1 Hz, 2H), 2.81-2.71 (m, 2H), 1.91-1.79 (m, 2H), 1.55 (q, J=10.9, 9.2 Hz, 2H).

### Example 20: Synthesis of compound 23

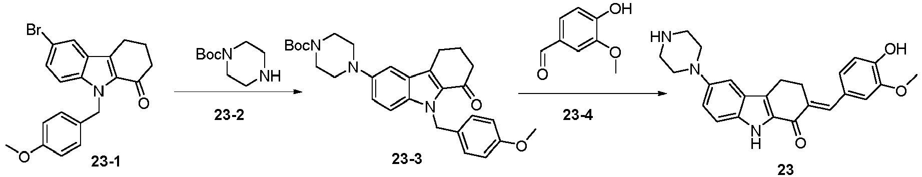

Under a nitrogen atmosphere, compound **23-1** (200 mg, 0.520 mmol), cesium carbonate (508 mg, 1.560 mmol), catalyst EPhosPdG₄(47 mg, 0.052 mmol), ligand EPhos (28 mg, 0.052 mmol) and a solution of compound **23-2** (116 mg, 0.624 mmol) in 1,4-dioxane (5 mL) were added at room temperature. Under a nitrogen atmosphere, the reaction solution was stirred at 100 °C for 4 hours and then quenched with water, extracted with ethyl acetate (3 × 10 mL), washed with saturated saline water (10 mL), dried over anhydrous sodium sulfate, concentrated, and the crude product was separated by silica gel column chromatography (petroleum ether: ethyl acetate=1:1)to obtain a white solid compound **23-3** (182 mg, 71.42%).ESI/MS (m/z): 490.3 [M+H]⁺.

Concentrated sulfuric acid (0.5 mL, 9.381 mmol) was added to a mixture of compounds **23-3** (150 mg, 0.286 mmol) and **23-4** (56 mg, 0.367 mmol) in methanol (5 mL) at room temperature. The reaction solution was stirred overnight at 65 °C, and then adjused to pH 7 with a saturated sodium bicarbonate aqueous solution, extracted with ethyl acetate (3 × 20 mL), washed with saturated salt water (20 mL), dried over anhydrous sodium sulfate, concentrated, and the crude product was separated b ya reverse phase preparation column (mobile phase A: water (10 mmol/L ammonium bicarbonate), mobile phase B: acetonitrile) to obtain a light yellow solid compound **23** (10.1 mg, 8.17%). ESI/MS (m/z): 404.0 [M+H]⁺. ¹H NMR (400 MHz, DMSO-d₆): δ 11.47 (s, 1H), 7.55 (s, 1H), 7.29 (d, *J*=9.0 Hz, 1H), 7.15 (dd, J=9.0, 2.0 Hz, 1H), 7.10 (d, .7=1.5 Hz, 1H), 7.03 (d, J=1.6 Hz, 1H), 7.01 (s, 1H), 6.86 (d, J=8.1 Hz, 1H), 3.82 (s, 3H), 3.21 (t, *J*=*5.8* Hz, 2H), 2.98 (t, J=4.6 Hz, 6H), 2.86 (s, 4H).

### Example 21: Synthesis of compound 24

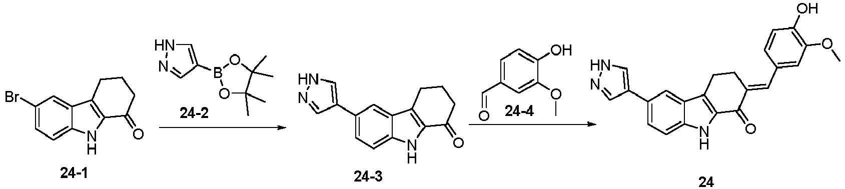

Under a nitrogen atmosphere, potassium acetate (371 mg, 3.786 mmol) was added to a solution of compounds **24-1** (500 mg, 1.893 mmol), **24-2** (734 mg, 3.786 mmol), catalyst Pd(dppf)Cl₂CH₂Cl₂ (231 mg, 0.284 mmol) in 1,4-dioxane (10 mL) and water (2 mL) at room temperature. Under a nitrogen atmosphere, the reaction solution was stirred overnight at 100 °C, then quenched with water, extracted with ethyl acetate (3 × 20 mL), washed with saturated salt water (20 mL), dried over anhydrous sodium sulfate and concentrated, and the crude product was separated by silica gel column chromatography (petroleum ether: ethyl acetate=1:1) to obtain a brown solid compound **24-3** (100 mg, 21.02%). ESI/MS (m/z): 252.2 [M+H]⁺.

Concentrated sulfuric acid (0.5 mL, 9.381 mmol) was added to a solution of compounds **24-3**(100 mg, 0.398 mmol) and **24-4** (72 mg, 0.478 mmol) in methanol (5 mL) at room temperature. The reaction solution was stirred overnight at 65 °C, and then adjusted to pH 8 with a saturated sodium bicarbonate aqueous solution. After concentration, the crude product was separated by reverse phase preparation column (mobile phase A: water (10 mmol/L ammonium bicarbonate), mobile phase B: acetonitrile) to obtain a brown compound **24** (12.2 mg, 7.80%). ESI/MS (m/z): 386.1 [M+H]⁺. ¹HNMR (400 MHz, DMSO-d₆): δ 12.86 (s, 1H), 11.69 (s, 1H), 9.41 (s, 1H), 8.04 (s, 2H), 7.90 (s, 1H), 7.61 (dd, J=8.7, 1.6 Hz, 1H), 7.59 (s, 1H), 7.40 (d, J=8.6 Hz, 1H), 7.13 (d, J=1.6 Hz, 1H), 7.04 (dd, J=8.2, 1.5 Hz, 1H), 6.88 (d, *J*=8.1 Hz, 1H), 3.83 (s, 3H), 3.26 (t, J=5.76 Hz, 2H), 3.07 (t, *J*=6.2 Hz, 2H).

### Example 22: Synthesis of compound 25

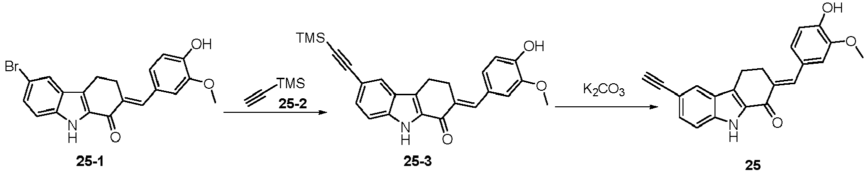

Triethylamine (228.6 mg, 2.259 mmol) and **25-2** (222 mg, 2.259 mmol) were added to a mixture of compound **25-1**(300 mg, 0.753 mmol), Pd(dppf)Cl₂CH₂Cl₂(123 mg, 0.151 mmol) and cuprous iodide (14 mg, 0.075 mmol) in DMF (5 mL) at room temperature under a nitrogen atmosphere. The reaction solution was stirred at 80 °C for 2 hours, then quenched with water (1 mL), extracted with ethyl acetate (3 × 10 mL), washed with saturated salt water (15 mL), dried over anhydrous sodium sulfate, and concentrated. The crude product was separated by silica gel column (petroleum ether: ethyl acetate=1:1) to obtain a yellow solid compound **25-3** (150 mg, 47.92%). ESI/MS (m/z): 416.0 [M+H]⁺.

Potassium carbonate (100 mg, 0.722 mmol) was added to a mixture of compound **25-3** (150 mg, 0.361 mmol) in DMF (5 mL) at room temperature. The reaction solution was stirred at 60 °C for 2 hours, then quenched with 1 mL of water, extracted with ethyl acetate (3 × 10 mL), and the organic phase was washed with saturated salt water (15 mL), dried over anhydrous sodium sulfate and concentrated, and the crude product was separated by reverse phase preparation column to obtain a yellow solid compound **25** (20.3 mg, 16.38%). ESI/MS (m/z): 344.0 [M+H]⁺. ¹H NMR (400 MHz, DMSO-d₆): δ 11.93 (s, 1H), 7.88 (d, J=1.2 Hz, 1H), 7.59 (d, *J*=1.9 Hz, 1H), 7.42 (d, J=8.6 Hz, 1H), 7.38 (dd, *J*=8.6, 1.4 Hz, 1H), 7.12 (d, *J*=1.7 Hz, 1H), 7.04 (dd,J=8.3, 1.5 Hz, 1H), 6.86 (d, J=8.2 Hz, 1H), 4.03 (s, 1H), 3.82 (s, *3H),* 3.24 (t, *J*=5.8 Hz, 2H) 3.04 (t, *J*=6.3 Hz, 2H).

### Example 23: Synthesis of compound 26

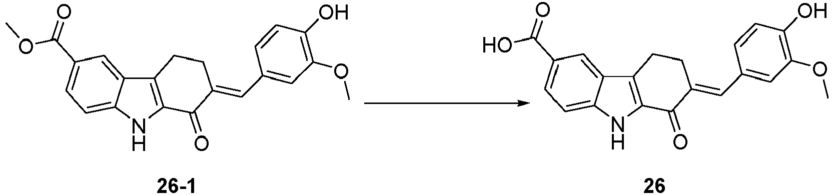

Lithium hydroxide (3 mg, 0.130 mmol) was added to a solution of compound **26-1** (10 mg, 0.026 mmol) in methanol (1 mL) at room temperature. The reaction solution was stirred overnight at 65 °C, and then adjusted to pH 8 with2M hydrochloric acid, concentrated, and the crude product was separated by reverse phase preparation column (mobile phase A: water (0.05% formic acid), mobile phase B: acetonitrile) to obtain a brown solid compound **26** (2 mg, 20.77%).ESI/MS (m/z): 364.0 [M+H]⁺.

### Example 24: Synthesis of compound 27

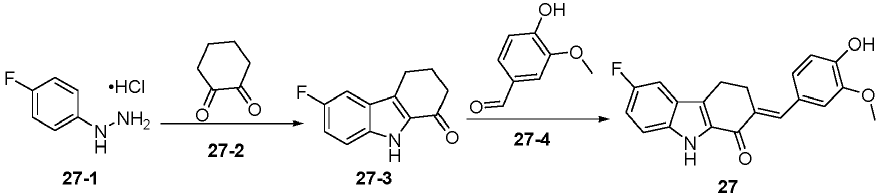

Concentrated hydrochloric acid (0.3 mL, 9.874 mmol) was added to a 12 mL aqueous solution of compounds **27-1**(300 mg, 1.845 mmol) and **27-2** (248 mg, 2.214 mmol) at room temperature. The reaction solution was stirred overnight at 120 °C. The obtained solid was collected and washed with water (30 mL) to obtain a brown solid compound **27-3** (180 mg, 48.01%). ESI/MS (m/z): 204.0 [M+H]⁺.¹HNMR (400 MHz, DMSO-d₆): δ 11.69 (s, 1H), 7.45 (dd,J=9.5, 2.2 Hz, 1H), 7.40 (dd, J=9.0, 4.5 Hz, 1H), 7.17 (td, J=9.2, 2.3 Hz, 1H), 2.92 (t, J=5.9 Hz, 2H), 2.62-2.53 (m, 2H), 2.15 (p, J=6.0 Hz, *2H).*

Concentrated sulfuric acid (0.3 mL, 5.628 mmol) was added to a mixture of compounds **27-**3(180 mg, 0.886 mmol) and **27-4** (594 mg, 5.302 mmol) in methanol (3 mL) at room temperature. The reaction solution was stirred overnight at 65 °C, and then adjusted to pH 8 with a saturated sodium bicarbonate aqueous solution. After concentration, the crude product was separated by reverse phase preparation column (mobile phase A: water (0.1% formic acid), mobile phase B: acetonitrile) to obtain a yellow solid compound **27** (20.4 mg, 6.83%). ESI/MS (m/z): 338.0 [M+H]⁺. ¹HNMR (400 MHz, DMSO-d₆): δ 11.83 (s, 1H), 9.48 (s, 1H), 7.59 (s, 1H), 7.48 (dd, *J*=*9.5,* 2.5 Hz, 1H), 7.42 (dd, *J*=9.0, 4.5 Hz, 1H), 7.19 (td, *J*=9.2, 2.6 Hz, 1H), 7.12 (d, *J*=1.8 Hz, 1H), 7.04 (dd, *J*=8.3, 1.7 Hz, 1H), 6.87 (d, *J*=8.1 Hz, 1H), 3.82 (s, 3H), 3.27-3.20 (m, 2H), 3.02 1 *(t, J*=*6.3* Hz, 2H).

### Example 25: Synthesis of compound 28

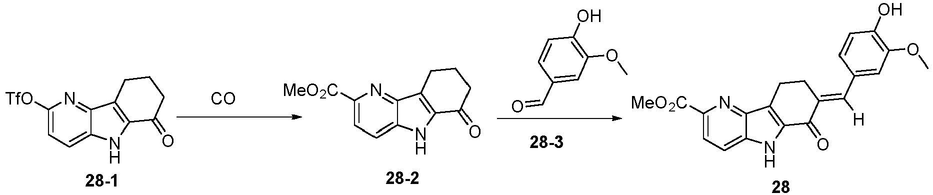

Triethylamine (0.19 mL, 1.347 mmol) was added to compound **28-1**(150 mg, 0.449 mmol) and catalyst Pd(dppf)Cl₂.CH2Cl2 (73 mg, 0.090 mmol) in methanol (20 mL) at room temperature. Under a carbon monoxide gas atmosphere, the reaction solution was stirred overnight at 80 °C, then concentrated, and the crude product was separated by silica gel column chromatography (dichloro: methanol=20: 1) to obtain a light yellow solid compound **28-2** (70 mg, 63.87%). ESI/MS (m/z): 245.0 [M+H]⁺. ¹HNMR (400 MHz, DMSO-d₆): δ 12.19 (s, 1H), 8.02 (d, *J*=8.7 Hz, 1H), 7.92 (d, J=8.7 Hz, 1H), 3.91 (s, 3H), 3.05 (t, J=5.7 Hz, 2H), 2.66 (t, *J*=6.2 Hz, 2H), 2.21 (p, *J*=5.8 Hz, 2H).

Concentrated sulfuric acid (0.3 mL) was added to a mixture of compounds **28-2** (50 mg, 0.205 mmol) and **28-3** (37 mg, 0.246 mmol) in methanol (3 mL) at room temperature. The reaction solution was stirred overnight at 65 °C, and then adjusted to pH 8 with a saturated sodium bicarbonate aqueous solution. After concentration, the crude product was separated by a reverse phase preparation column (mobile phase A: water (0.1% formic acid), mobile phase B: acetonitrile) to obtain a yellow solid compound **28** (12.5 mg, 16.14%). ESI/MS (m/z): 379.0 [M+H]⁺. ¹HNMR (400 MHz, DMSO-d₆): δ 12.07 (s, 1H), 8.04 (d, J=8.6 Hz, 1H), 7.94 (d, *J*=8.7 Hz, 1H), 7.64 (s, 1H), 7.15 (s, 1H), 7.08 (d, J=8.3 Hz, 1H), 6.89 (d, J=8.2 Hz, 1H), 3.91 (s, 3H), 3.83 (s, 3H), 3.31 (s, 2H), 3.13 (d, J=6.3 Hz, 2H).

### Example 26: Synthesis of compound 29

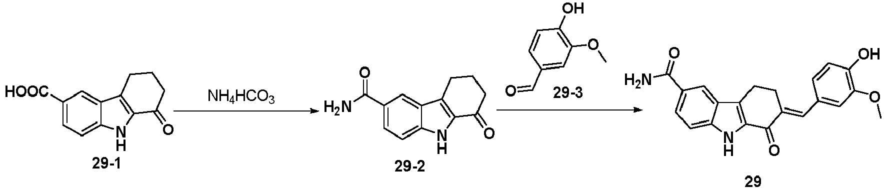

Triethylamine (397 mg, 3.927 mmol) and HATU (746.42 mg, 1.963 mmol) were added to a solution of compound **29-1** (300 mg, 1.309 mmol) and ammonium bicarbonate (310 mg, 3.927 mmol) in DMF (10 mL) at room temperature. The reaction solution was stirred at room temperature for 2 hours, then quenched with saturated ammonium chloride aqueous solution, extracted with ethyl acetate, dried over anhydrous sodium sulfate, and concentrated. The crude product was separated by silica gel column chromatography (petroleum ether: ethyl acetate=1:1) to obtain a gray solid compound **29-2** (150 mg, 50.22%). ESI/MS (m/z): 226.9 [M-H]⁻. ¹HNMR (400 MHz, DMSO-d₆): δ 11.82 (s, 1H), 8.31 (s, 1H), 7.92 (s, 1H), 7.84 (s, 1H), 7.40 (d, J=8.7 Hz, 1H), 7.20 (s, 1H), 2.99 (t, J=5.9 Hz, 2H), 2.64-2.55 (m, 2H), 2.19 (q, J=6.0 Hz, 2H).

Concentrated sulfuric acid (0.3 mL, 5.628 mmol) was added to a solution of compounds **29-2** (150 mg, 0.657 mmol) and **29-3** (120 mg, 0.788 mmol) in methanol (3 mL) at room temperature. The reaction solution was stirred overnight at 65 °C, and then adjusted to pH 8 with a saturated sodium bicarbonate aqueous solution. After concentration, the crude product was separated by reverse phase preparation column (mobile phase A: water (0.1% formic acid), mobile phase B: acetonitrile) to obtain a brown solid compound **29** (8.5 mg, 3.57%). ESI/MS (m/z): 361.0 [M-H]⁻. ¹HNMR (400 MHz, DMSO-d₆): δ 11.97 (s, 1H), 9.50 (s, 1H), 8.33 (s, 1H), 7.95 (s, 1H), 7.88 (dd, *J*=8.7, 1.4 Hz, 1H), 7.60 (s, 1H), 7.44 (d, J=8.7 Hz, 1H), 7.22 (s, 1H), 7.14 (d, J=1.8 Hz, 1H), 7.05 (dd, J=8.3, 1.6 Hz, 1H), 6.88 (d, *J*=8.1 Hz, 1H), 3.83 (s, 3H), 3.28 (t, J=5.78 Hz, 2H, 3.08 (t, J=6.3 Hz, 2H).

### Example 27: Synthesis of compound 30

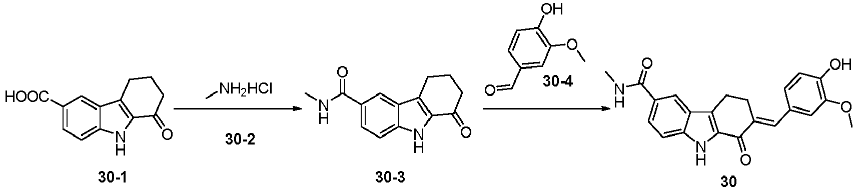

DIEA (1.14 mL, 6.545 mmol) and HATU (597 mg, 1.571 mmol) were added to a solution of compounds **30-1** (300 mg, 1.309 mmol) and **30-2** (106 mg, 1.571 mmol) in DMF (3 mL) at room temperature. The reaction solution was stirred at room temperature for 2 hours, then quenched with water, extracted with ethyl acetate (3 × 10 mL), concentrated, and the crude product was separated bysilica gel column chromatography (dichloromethane: methanol=10:1) to obtain a brown compound **30-3** (180 mg, 56.77%). ESI/MS (m/z): 243.0 [M+H]⁺. ¹HNMR (400 MHz, DMSO-d₆): δ 11.84 (s, 1H), 8.39 (d, J=4.4 Hz, 1H), 8.25 (s, 1H), 7.83 (dd, J=8.7, 1.4 Hz, 1H), 7.43 (d, J=8.7 Hz, 1H), 2.99 (t, J=5.9 Hz, 2H), 2.82 (d, J=4.5 Hz, 3H), *2.66-2.55* (m, 2H), 2.18 (p, J=5.9 Hz, 2H).

Concentrated sulfuric acid (0.3 mL, 5.629 mmol) was added to a solution of compounds **30-3(180** mg, 0.743 mmol) and **30-4** (135 mg, 0.892 mmol) in methanol (3 mL) at room temperature. The reaction solution was stirred overnight at 80 °C, and then adjusted to pH 8 with a saturated sodium bicarbonate aqueous solution. After concentration, the crude product was separated by reverse phase preparation column (mobile phase A: water (0.1% formic acid), mobile phase B: acetonitrile) to obtain a yellow solid compound **30** (11.3 mg, 4.04%). ESI/MS (m/z): 377.1 [M+H]⁺. ¹HNMR (400 MHz, DMSO-d₆): δ 11.96 (s, 1H), 9.47 (s, 1H), 8.39 (d, J=4.6 Hz, 1H), 8.25 (s, 1H), 7.83 (d, J=10.4 Hz, 1H), 7.59 (s, 1H), 7.42 (s, 1H), 7.13 (s, 1H), 7.05 (d, J=10.1 Hz, 1H), 6.87 (d, J=8.1 Hz, 1H), 3.83 *(s,* 3H), 3.27 (t, J=6.4 Hz, 2H), 3.07 (t, *J*=6.3 Hz, 2H), 2.80 (d, *J*=4.5 Hz, 3H).

### Example 28: Synthesis of compound 31

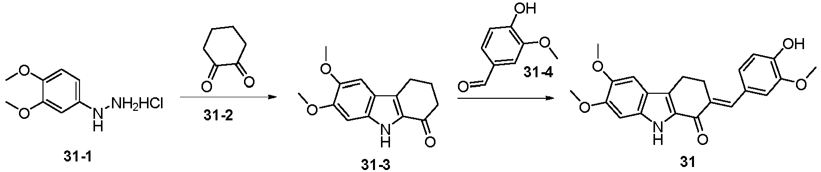

Concentrated hydrochloric acid(5 mL)was added to an aqueous (100 mL)solution of compounds **31-1** (1.2 g, 5.945 mmol) and **31-2** (1 g, 8.918 mmol) at room temperature. The reaction solution was refluxed overnight at 120 °C. It was then cooled down to room temperature, filtered. The obtained solid was collected, washed with ice water, and subjected to silica gel column chromatography (petroleum ether: ethyl acetate=1:1)to obtain a yellow solid compound **31-3**(120 mg, 8.23%). ESI-MS (m/z): 245.95 [M+H]⁺.

Concentrated sulfuric acid (0.5 mL, 9.381 mmol) was added to a solution of compounds **31-3**(120 mg, 0.489 mmol) and **31-4** (89 mg, 0.587 mmol) in methanol (5 mL) at room temperature. The reaction solution was stirred at 65 °C overnight, and then adjusted to pH 8 with a saturated sodium carbonate aqueous solution. After concentration, the crude product was separated by reverse phase preparation column to obtain a yellow solid compound 31 (74.0 mg, 38.31%). ESI/MS (m/z): 380.0 [M+H]⁺. ¹H NMR (400 MHz, DMSO-d₆): δ 11.49 (s, 1H), 9.42 (s, 1H), 7.54 (s, 1H), 7.10 (s, 2H), 7.01 (d, J=8.0 Hz, 1H), 6.86 (d, J=8.0 Hz, 1H), 6.85 (s, 1H), 3.82 (s, 6H), 3.79 (s, 3H), 3.19 (t, J=6.4 Hz, 2H), 2.98 (t, J=6.3 Hz, 2H).

### Example 29: Synthesis of compound 32

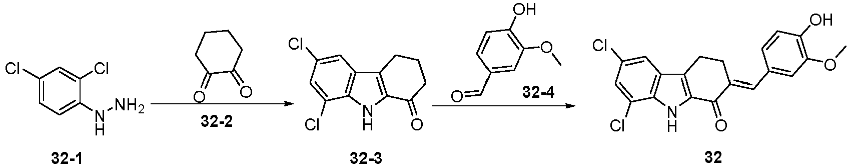

Concentrated hydrochloric acid (0.5 mL, 16.456 mmol) was added to an aqueous (20 mL) solution of compounds **32-1**(300 mg, 1.695 mmol) and **32-2** (228 mg, 2.034 mmol) at room temperature. The reaction solution was stirred overnight at 120 °C. The obtained solid was collected, washed with water (30 mL) to obtain a brown solid compound **32-3** (170 mg, 39.48%). ESI/MS (m/z): 255.1 [M+H]⁺. ¹H NMR (400 MHz, DMSO-d₆): δ 12.17 (s, 1H), 7.79 (s, 1H), 7.47 (s, 1H), 2.94 (d, *J*=11.7 Hz, 2H), 2.64-2.54 (m, 2H), 2.15 (p, J=5.9 Hz, 2H).

Concentrated sulfuric acid (0.3 mL, 5.629 mmol) was added to a solution of compounds **32-3(**170 mg, 0.669 mmol) and **32-4** (122 mg, 0.803 mmol) in methanol (3 mL) at room temperature. The reaction solution was stirred overnight at 65 °C, and then adjusted pH to 8 with a saturated sodium bicarbonate aqueous solution. After concentration, the crude product was separated by reverse phase preparation column (mobile phase A: water (0.1% formic acid), mobile phase B: acetonitrile) to obtain a yellow solid compound **32** (27 mg, 10.40%). ESI/MS (m/z): 388.1 [M+H]⁺. ¹HNMR (400 MHz, DMSO-d₆): δ 12.30 (s, 1H), 9.51 (s, 1H), 7.81 (s, 1H), 7.62 (s, 1H), 7.50 (d, J=1.6 Hz, 1H), 7.13 (s, 1H), 7.05 (d, J=8.1 Hz, 1H), 6.88 (d, J=8.1 Hz, 1H), 3.83 (s, 3H), 3.24 (t, J=5.8 Hz, 2H), 3.02 (t, *J*=6.2 Hz, 2H).

### Example 30: Synthesis of compound 33

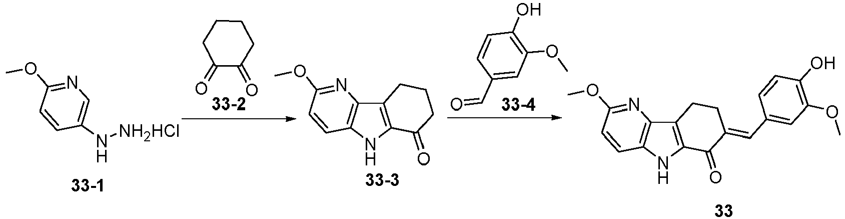

Concentrated hydrochloric acid (2.40 mL, 78.989 mmol) was added to a aqueous (100 mL) solution of compounds **33-1**(1.2 g, 6.833 mmol) and **33-2** (0.92 g, 8.200 mmol) at room temperature. The reaction solution was stirred overnight at 120 °C, concentrated, and the crude product was separated by silica gel column chromatography (petroleum ether: ethyl acetate=1:1) to obtain a yellow solid compound **33-3** (400 mg, 27.07%). ESI/MS (m/z): 217.1 [M+H]⁺. ¹HNMR (400 MHz, DMSO-d₆) δ 11.46 (s, 1H), 7.73 (d, J=8.9 Hz, 1H), 6.79 (d, *J*=8.9 Hz, 1H), 3.91 (s, 3H), 2.94 (t, J=6.0 Hz, 2H), 2.63-2.54 (m, 2H), 2.14 (p, *J*=6.2 Hz, 2H).

Concentrated sulfuric acid (0.40 mL, 7.511 mmol) was added to a solution of compounds **33-3**(400 mg, 1.850 mmol) and **33-4** (309 mg, 2.035 mmol) in methanol (5 mL) at room temperature. The reaction solution was stirred overnight at 65 °C, and then adjusted to pH 8 with a saturated sodium bicarbonate aqueous solution. After concentration, the crude product was separated by reverse phase preparation column (mobile phase A: water (10 mmol/L ammonium bicarbonate), mobile phase B: acetonitrile) to obtain a yellow solid compound **33** (110 mg, 16.43%). ESI/MS (m/z): 351.0 [M+H]⁺. ¹H NMR (400 MHz, DMSO-d₆): δ 11.87 (s, 1H), 9.48 (s, 1H), 7.75 (d, J=8.9 Hz, 1H), 7.61 (s, 1H), 7.13 (s, 1H), 7.03 (s, 1H), 6.89 (d, *J*=8.1 Hz, 1H), 6.81 (d, *J*=8.9 Hz, 1H), 3.91 (s, 3H), 3.84 (s, 3H), 3.23 (t, J=5.7 Hz, 2H), *3.01* (t, *J*=6.2 Hz, 2H).

### Example 31: Synthesis of compound 34

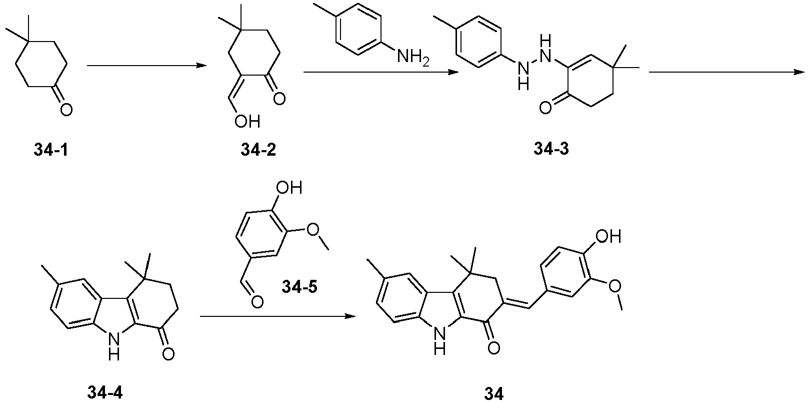

Sodium ethanol (1 g, 15.848 mmol) was added to a solution of compound **34-1** (1 g, 7.924 mmol) and ethyl formate (880 mg, 11.886 mmol) in 1,4-dioxane (10 mL) at 0 °C. The reaction solution was stirred at room temperature for 1 hour, and then adjusted to pH 2 with2 M hydrochloric acid, extracted with ethyl acetate, washed with saturated salt water, dried over anhydrous sodium sulfate, and concentrated. The crude product was separated by silica gel column chromatography (petroleum ether: ethyl acetate=5:1) to obtain a brown oily liquid **34-2** (600 mg, 49.10%). ESI/MS (m/z): 153.2 [M-H]⁻.

An aqueous (10 mL) solution of sodium nitrite (643 mg, 9.332 mmol) was added to an aqueous (20 mL) solution of compound p-methylaniline (500 mg, 4.666 mmol) and concentrated hydrochloric acid (3.7 mL, 123.416 mmol) at 0 °C. A mixed solution of compound **34-2** (719 mg, 4.666 mmol) and sodium acetate (842 mg, 10.265 mmol) in methanol and water (40 mL, 1:1) was added to the reaction solution at 0 °C. The reaction solution was stirred at room temperature for additional 2 hours. The obtained solid was collected and washed with water (30 mL) to obtain a yellow solid compound **34-3** (255 mg, 22.37%). ESI-MS (m/z): 245.0 [M+H]⁺.

Concentrated sulfuric acid, MeOH (0.1 mL) was added to a solution of compound **34-3** (250 mg, 1.023 mmol) and acetic acid (0.1 mL) in methanol (1 mL) at room temperature, the reaction solution was stirred at 65 °C for 24 hours, then adjusted to pH 8 with a saturated sodium bicarbonate aqueous solution, extracted with ethyl acetate (2 × 10 mL), and concentrated. The crude product was separated by silica gel column chromatography (petroleum ether: ethyl acetate=3:1) to obtain a yellow solid compound **34-4** (88 mg, 37.84%).ESI/MS (m/z): 228.3 [M+H]⁺.

Concentrated sulfuric acid (0.2 mL, 3.752 mmol) was added to a mixed solution of compound **34-4** (80 mg, 0.352 mmol) and **34-5** (64 mg, 0.422 mmol) in methanol (2 mL) at room temperature. The reaction solution was stirred overnight at 65 °C, and then adjusted to pH 8 with a saturated sodium bicarbonate aqueous solution. After concentration, the crude product was separated by reverse phase preparation column (mobile phase A: water (10 mmol/L ammonium bicarbonate), mobile phase B: acetonitrile) to obtain a yellow solid compound **34** (28 mg, 22.01%). ESI/MS (m/z): 362.1 [M+H]⁺. ¹H NMR (400 MHz, DMSO-d₆): δ 11.60 (s, 1H), 9.47 (s, 1H), 7.66 (s, 1H), 7.61 (s, 1H), 7.33 (d, J=8.5 Hz, 1H), 7.16-7.11 (m, 1H), 7.11 (d, *J*=1.6 Hz, 1H), 7.04 (dd, J=8.2, 1.5 Hz, 1H), 6.87 (d, J=8.1 Hz, 1H), 3.82 (s, 3H), 3.07 (s, 2H), 2.39 (s, 3H), 1.47 (s, 6H).

### Example 32: Synthesis of compound 35

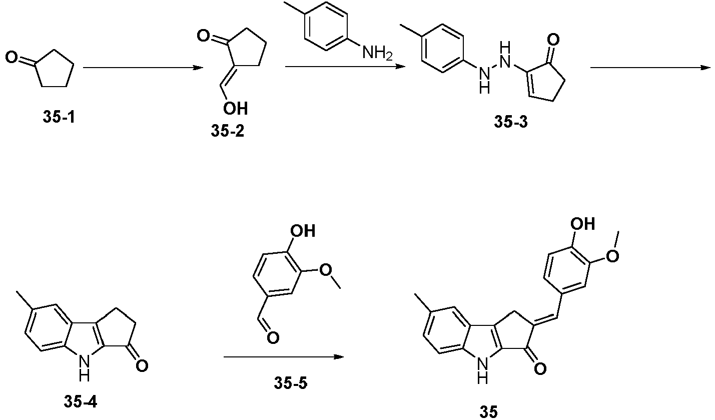

Sodium hydride (1.71 g, 71.328 mmol) was added to a solution of compound **35-1** (2 g, 23.776 mmol) and ethyl formate (6.28 g, 71.328 mmol) in toluene (23 mL) at 0 °C. The reaction solution was stirred at room temperature for 2 hours, and then adjusted to pH 2 with 2 M hydrochloric acid, extracted with ethyl acetate (3 × 50mL) and concentrated to obtain a colorless oily liquid **35-2** (1.5 g, 56.26%). ESI/MS (m/z): 113.1 [M+H]⁺.

An aqueous (10 mL) solution of sodium nitrite (615 mg, 8.918 mmol) was added to a aqueous (20 mL) solution of compound p-methylaniline (477 mg, 4.459 mmol) and concentrated hydrochloric acid (1 mL) at 0 °C. A mixed solution of compound **35-2** (500 mg, 4.459 mmol) and sodium acetate (804 mg, 9.810 mmol) in methanol and water (40 mL, 1:1) was added to the above mixture at 0 °C. The reaction solution was stirred at 0 °C for 1 hour. The obtained solid was collected and washed with water (3 × 50 mL) to obtain a yellow solid compound **35-3** (800 mg, 88.70%).ESI/MS (m/z): 203.0 [M+H]⁺.

Concentrated sulfuric acid (0.4 mL, 7.504 mmol) was added to an solution of compound **35-3** (400 mg, 1.978 mmol) in acetonitrile (10 mL) at room temperature. The reaction solution was stirred at 65 °C for 2 hours, and then adjusted to pH 7 with a saturated sodium bicarbonate aqueous solution, extracted with ethyl acetate (3 × 30 mL), and concentrated. The crude product was separated by silica gel column chromatography (petroleum ether: ethyl acetate=5:1) to obtain a yellow solid compound **35-4** (150 mg, 40.95%). ESI/MS (m/z): 186.2 [M+H]⁺.

Concentrated sulfuric acid (0.4 mL, 7.504 mmol) was added to a mixed solution of compounds **35-4** (150 mg, 0.810 mmol) and **35-5** (147 mg, 0.972 mmol) in methanol at room temperature. The reaction solution was stirred overnight at 65 °C, and then adjusted to pH 8 with a saturated sodium bicarbonate aqueous solution. After concentration, the crude product was separated by reverse phase preparation column (mobile phase A: water (10 mmol/L ammonium bicarbonate), mobile phase B: acetonitrile) to obtain a yellow solid compound **35** (22 mg, 8.51%). ESI/MS (m/z): 320.0 [M+H]⁺. ¹H NMR (400 MHz, DMSO-d₆): δ 11.72 (s, 1H), 9.62 (s, 1H), 7.57 (s, 1H), 7.36 (d, J=8.5 Hz, 1H), 7.32 (s, 1H), 7.30 (s, 1H), 7.23 (d, *J*=8.3 Hz, 1H), 7.20 (d, J=8.5 Hz, 1H), 6.90 (d, J=8.1 Hz, 1H), 3.99 (s, 2H), 3.89 (s, 3H), 2.41 (s, 3H).

### Example 33: Synthesis of compound 36

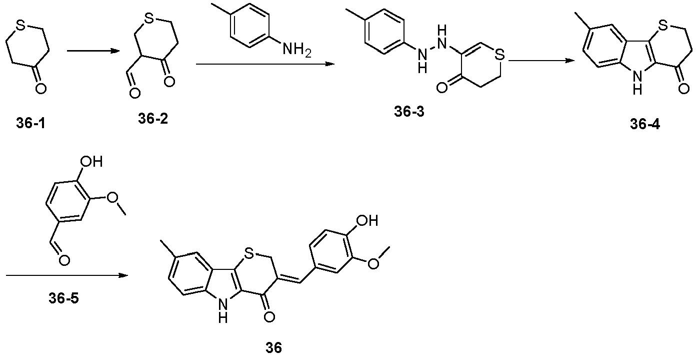

Sodium methoxide (0.47 g, 8.607 mmol) was added to a mixture of compound **36-1** (1 g, 8.607 mmol) and ethyl formate (0.64 g, 8.607 mmol) in dioxane (10 mL) at 0 °C. The reaction solution was stirred at room temperature for 2 hours, and then adjusted to pH 7 with2 M hydrochloric acid, extracted with ethyl acetate (3 × 30 mL), washed with saturated salt water (30 mL), dried over anhydrous sodium sulfate, and concentrated. The crude product was separated by silica gel column chromatography (petroleum ether: ethyl acetate=5:1) to obtain a light brown solid compound **36-2** (490 mg, 39.48%). ESI/MS (m/z): 145.0 [M+H]⁺.

An aqueous (10 mL) solution of sodium nitrite (431 mg, 6.242 mmol) was added to an aqueous (10 mL) solution of compound p-methylaniline (334 mg, 3.121 mmol) and concentrated hydrochloric acid (1.5 mL) at 0 °C, and a solution of compound **36-2** (450 mg, 3.121 mmol) and sodium acetate (563 mg, 6.242 mmol) in aqueous and methanol (20 mL, 1:1) was added. The reaction solution was stirred at room temperature for 2 hours. The obtained solid was collected and used with water (3 × 100 mL) to obtain a yellow solid compound **36-3** (180 mg, 24.61%). ESI/MS (m/z): 235.0 [M+H]⁺.

Concentrated sulfuric acid (0.2 mL, 3.005 mmol) was added to a solution of compound **36-3** (160 mg, 0.683 mmol) and acetic acid (0.1 mL) in methanol (1 mL) at room temperature. The reaction solution was stirred overnight at 65 °C. It was then concentrated, and separated by silica gel column chromatography (petroleum ether: ethyl acetate=5:1) to obtain a yellow solid compound **36-4** (100 mg, 67.40%). ESI-MS (m/z): 218.2 [M+H]⁺.

Concentrated sulfuric acid (0.1 mL, 1.876 mmol) was added to a mixture of compounds **36-4** (80 mg, 0.390 mmol) and **36-5** (71 mg, 0.468 mmol) in methanol (1 mL) at room temperature. The reaction solution was stirred overnight at 65 °C, and then adjusted to pH 8 with a saturated sodium bicarbonate aqueous solution. After concentration, the crude product was separated by reverse phase preparation column (mobile phase A: water (0.1% formic acid), mobile phase B: acetonitrile) to obtain a yellow solid compound **36** (8.1 mg, 5.91%). ESI/MS (m/z): 352.0 [M+H]⁺. ¹HNMR (400 MHz, DMSO-d₆): δ 11.85 (s, 1H), 7.58 (s, 1H), 7.33 (d, *J*=8.7 Hz, 1H), 7.31 (s, 1H), 7.22-7.16 (m, 1H), 7.11 (d, J=1.5 Hz, 1H), 7.06-7.01 (m, 1H), 6.89 (d, *J*=8.1 Hz, 1H), 4.39 (s, 2H), 3.83 (s, 3H), 2.38 (s, 3H).

### Example 34: Synthesis of compound 37

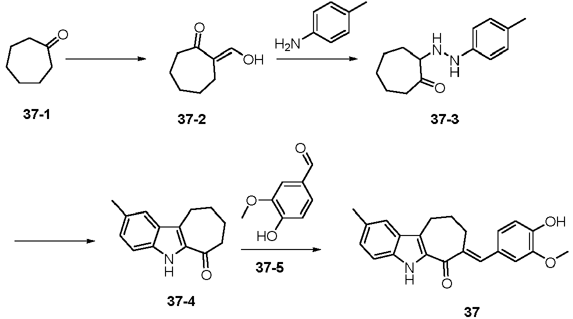

Sodium ethanol (1.21 g, 17.830 mmol) was added to a mixture of compound **37-1** (1 g, 8.915 mmol) and ethyl formate (1.32 g, 17.830 mmol) in dioxane (10 mL) at 0 °C. The reaction solution was stirred at 0 °C for additional 1 hour, and then adjusted to pH 7 with 2 M hydrochloric acid, extracted with ethyl acetate, washed with saturated salt water, dried over anhydrous sodium sulfate, and concentrated. The crude product was separated by silica gel column chromatography (petroleum ether: ethyl acetate=10:1) to obtain a light brown solid oily liquid **37-2** (700 mg, 56.01%). ESI/MS (m/z): 141.1 [M+H]⁺.

An aqueous (5mL) solution of sodium nitrite (590 mg, 8.560 mmol) was added to an aqueous (20mL) solution of compound 4-methylaniline (458 mg, 4.280 mmol) and concentrated hydrochloric acid (1.50 mL, 48.382 mmol) at 0 °C. Maintain 0 °C and a mixed solution of compound **37-2** (600 mg, 4.280 mmol) and sodium acetate (772 mg, 9.416 mmol) in methanol and water(40 mL, 1:1) was added to the mixture. The reaction solution was stirred at room temperature for additional 2 hours. The obtained solid was collected, washed with water (3 × 50 mL), and separated by silica gel column chromatography (petroleum ether: ethyl acetate=2:1) to obtain a brown solid compound **37-3** (300 mg, 30.43%). ESI/MS (m/z): 231.0 [M+H]⁺.

Concentrated sulfuric acid (0.5 mL, 10.944 mmol) was added to a solution of compound **37-3** (280 mg, 1.216 mmol) and acetic acid (1 mL, 24.320 mmol) in methanol (5 mL) at room temperature. The reaction solution was stirred overnight at 65 °C, and then adjusted to pH 8 with a saturated sodium bicarbonate aqueous solution, extracted with ethyl acetate, washed with saturated salt water, and concentrated. The crude product was separated by silica gel column chromatography (petroleum ether: ethyl acetate=1:1) to obtain a brown solid compound **37-4** (135 mg, 52.06%). ESI-MS (m/z): 214.2 [M+H]⁺. ¹HNMR (400 MHz, DMSO-d₆): δ 11.19 (s, 1H), 7.43 (s, 1H), 7.28 (d, J=8.4 Hz, 1H), 7.14-7.05 (m, 1H), 3.12-2.96 (m, 2H), 2.79-2.68 (m, 2H), 2.37 (s, 3H), 1.96 (p, J=6.1 Hz, 2H), 1.86 (p, J=5.7, 5.0 Hz, 2H).

Concentrated sulfuric acid (0.3 mL, 5.628 mmol) was added to a mixtureof compounds **37-4** (100 mg, 0.469 mmol) and **37-5** (85 mg, 0.563 mmol) in methanol (3 mL) at room temperature. The reaction solution was stirred overnight at 65 °C, and then adjusted to pH 8 with a saturated sodium bicarbonate aqueous solution. After concentration, the crude product was separated by reverse phase preparation column (mobile phase A: water (10 mmol/L ammonium bicarbonate+0.1% ammonia water), mobile phase B: acetonitrile) to obtain a yellow solid compound **37** (32.6 mg, 19.81%). ESI/MS (m/z): 348.1 [M+H]⁺. ¹H NMR (400 MHz, DMSO-d₆): δ 11.27 (s, 1H), 9.38 (s, 1H), 7.51 (s, 1H), 7.44 (s, 1H), 7.32 (d, J=8.4 Hz, 1H), 7.12 (dd, *J*=8.4, 1.2 Hz, 1H), 7.07 (d, J=1.7 Hz, 1H), 6.98 (dd, J=8.2, 1.6 Hz, 1H), 6.87 (d, *J*=8.1 Hz, 1H), 3.83 (s, 3H), 3.11 (t, J=6.3 Hz, 2H), 2.90-2.81 (m, 2H) 2.39 (s, 3H), 2.12 (dt, *J*=11.9, 6.2 Hz, 2H).

### Example 35: Synthesis of compound 38

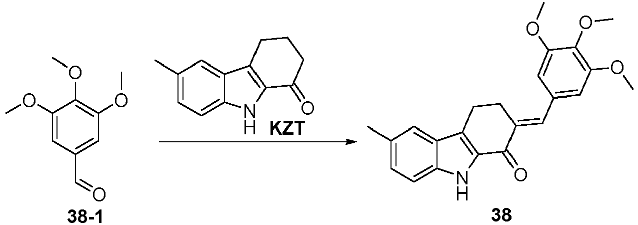

Potassium hydroxide (89.8 mg, 1.600 mmol) was added to a mixture of compound **38-1** (78.5 mg, 0.400 mmol) and **KZT** (159.4 mg, 0.800 mmol) in 1,4-dioxane (3 mL) at room temperature. The reaction was heated up to 100 °C and stirred for 2 hours. The reaction mixture was cooled down to room temperature, and the reaction was quenched with water. The obtained mixture was extracted with ethyl acetate (3 × 30 mL). The combined organic layer was dried over anhydrous sodium sulfate, filtered, and concentrated under reduced pressure. The crude product was separated and purified by silica gel column chromatography (ethyl acetate: petroleum ether=1:5) to obtain a yellow solid **38** (21.8mg, 14.44%). ESI/MS (m/z) 377.9 [M+H]⁺. ¹HNMR (400 MHz, DMSO-d₆) δ 11.62 (s, 1H), 7.57 (s, 1H), 7.45 (s, 1H), 7.32 (d, *J*=8.5 Hz, 1H), 7.17 (dd, J=8.4Hz, J=1.3Hz, 1H), 6.83 (s, 2H), 3.82 (s, 6H), 3.71 (s, 3H), 3.23 (t, *J*=4.8 Hz, 2H), 3.01 (t, *J*=6.2 Hz, 2H), 2.38 (s, 3H).

### Example 36: Synthesis of compound 39

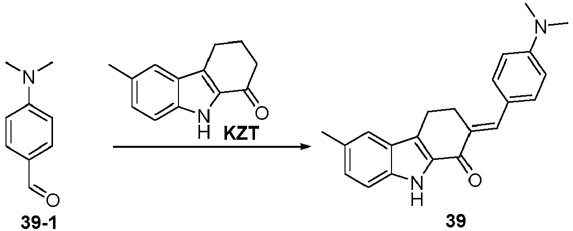

Potassium hydroxide (168.6 mg, 3.011 mmol) was added to a mixture of **KZT(**200.0 mg, 1.004 mmol) and compound **39-1** (149.8 mg, 1.004 mmol) in 1,4-dioxane (4 mL) at room temperature. The reaction was heated up to 100 °C for 6 hours. It was then quenched with water, and the obtained mixture was extracted with ethyl acetate (3 × 30 mL). The combined organic layers were washed with saturated salt water (5 mL), dried over anhydrous sodium sulfate, filtered, and concentrated under reduced pressure. The crude product was separated and purified by silica gel column chromatography (methanol: dichloromethane=1:20) to obtain an orange solid compound **39** (20.87 mg, 6.29%). ESI/MS (m/z) 330.9 [M+H]⁺.¹H NMR (400 MHz, CDCl3) δ 8.77 (s, 1H), 7.78 (s, 1H), 7.49-7.43 (m, 3H), 7.34 (d,J=8.5 Hz, 1H), 7.22 (dd, *J*=8.5, 1.4 Hz, 1H), 6.77 (d, J=8.4 Hz, 2H), 3.34 (t, *J*=6.4 Hz, 2H), 3.09-3.03 (m, 8H), 2.48 (s, 3H).

### Example 37: Synthesis of compound 40

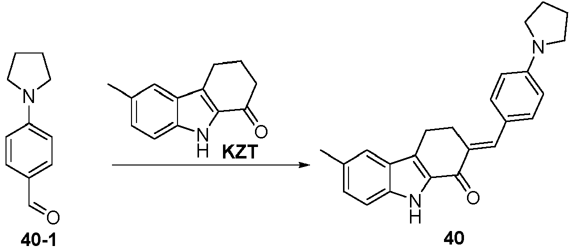

Potassium hydroxide (168.6 mg, 3.011 mmol) was added to a mixture of **KZT** (200.0 mg, 1.004 mmol) and **40-1** (175.9 mg, 1.004 mmol) in 1,4-dioxane (4 mL) at room temperature. The reaction was heated up to 100 °Cfor 6 hours. It was then quenched with water, and the obtained mixture was extracted with ethyl acetate (3 × 30 mL). The combined organic layers were washed with saturated salt water (5 mL), dried over anhydrous sodium sulfate, filtered, and concentrated under reduced pressure. The crude product was separated and purified by silica gel column chromatography (methanol: dichloromethane=1:20) to obtain an orange solid compound **40** (30.79 mg, 8.60%). ESI/MS (m/z) 357.0 [M+H]⁺. ¹H NMR (400 MHz, CDCl3) δ 8.88 (s, 1H), 7.79 (s, 1H), 7.47-7.45 (m, 3H), 7.34 (d,J=8.4 Hz, 1H), 7.21 (dd, J=8.4, 1.4 Hz, 1H), 6.62 (d, *J*=8.8 Hz, 2H), 3.40-3.33 (m, 6H), 3.06 (t, J=6.4 Hz, 2H), 2.48 (s, 3H), 2.09-2.02 (m, 4H).

### Example 38: Synthesis of compound 41

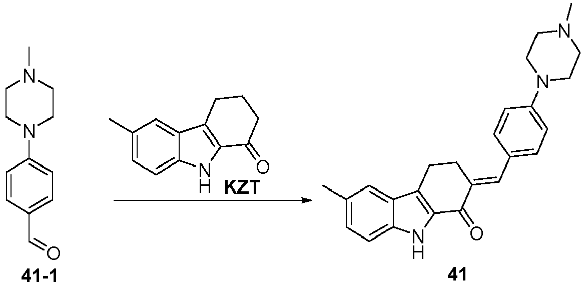

Potassium hydroxide (168.6 mg, 3.011 mmol) was added to a mixture of **KZT**(200.0 mg, 1.004 mmol) and **41-1** (205.1 mg, 1.004 mmol) in 1,4-dioxane (4 mL) at room temperature. The reaction was heated up to 100 °C for 6 hours. The reaction was quenched with water, and the obtained mixture was extracted with ethyl acetate (3 × 30 mL). The combined organic layers were washed with saturated salt water (5 mL), dried over anhydrous sodium sulfate, filtered, and concentrated under reduced pressure. The crude product was separated and purified by a silica gel column chromatography (methanol: dichloromethane=1:20) to obtain a deep yellow solid compound **41** (120 mg, 31.0%). ESI/MS (m/z) 385.9 [M+H]⁺. ¹H NMR (400 MHz, CDCl3) δ 8.94 (s, 1H), 7.77 (s, 1H), 7.45 (d, J=8.5 Hz, 3H), 7.35 (d, J=8.4 Hz, 1H), 7.23 (dd, *J*=8.5, 1.4 Hz, 1H), 6.97 (d, J=8.8 Hz, 2H), 3.40-3.26 (m, 6H), 3.06 (t, J=6.4 Hz, 2H), 2.67-2.54 (m, 4H), 2.48 (s, 3H), 2.39 (s, 3H).

### Biological Test Example 1 Verification of the Action Mechanism of Compounds

### 1. Verification of the dual target inhibition mechanism of compounds: (1) The inhibitory effect of compounds on the OCT4 protein in tumor stem cells (CSC)

### Test compounds: KZT, EKZT, KZT-A1 (compound 10), KZT-A3 (compound 11), KZT-A5 (compound 5), KZT-A6 (compound 2), and KZT-A7 (compound 12)

**Table 1: Test compounds and structural formulas thereof for inhibiting OCT4 protein**

| Compound Naming | Structural formula |
|---|---|
| KZT | 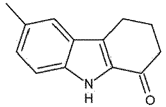 |
| EKZT | 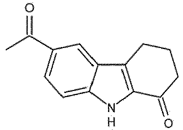 |
| KZT-A1 (Compound 10) | 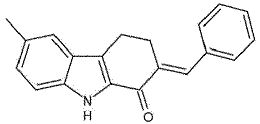 |
| KZT-A3 (Compound 11) | 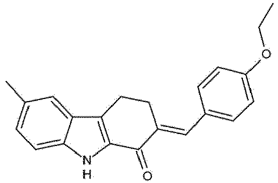 |
| KZT-A5 (Compound 5) | 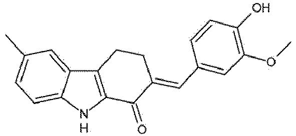 |
| KZT-A6 (Compound 2) | 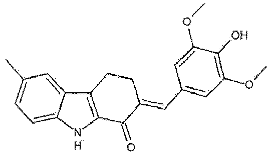 |
| KZT-A7 (Compound 12) | 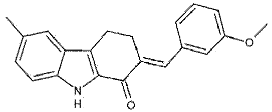 |

### 1. Test procedure

1) Cell source: HeLa cell line 3A11 with endogenous high expression of OCT4, constructed by the inventor's laboratory (Zhou Y et al. Endogenous authentic OCT4A proteins directly regulate FOS/AP-1 transcription in physiological cancer cells. Cell Death Dis. 2018, 9:585).
2) Cell culture and passage: All of the above cells were adherently cultured in complete culture medium [to a 6-cm culture dish (# 430166, Corning) or T75 culture bottle (# 3276, Corning) containing DMEM high-sugar basal medium (#SH30243.01B, HyClone) was added a final concentration of 10% FBS (# 1101-500, Shanghai Pufei) and Penicillin-Streptomycin dual antibody (# SV30010, HyClone)]. The culture dish (bottle) was placed in a cell culture incubator (# 3111, Thermo Fisher Scientific) at 37 °C, 5% CO₂, and saturated humidity. When passaging, the culture medium was aspirated, and they were washed twice with PBS phosphate buffer solution (# GNM-10944, Hangzhou Gino), then an appropriate amount of 0.25% trypsin-0.02% EDTA (# 25200-072, Gibco) was added. The culture dish (bottle) was shaken to make it uniformly cover the cells, and then placed under a phase contrast microscope for observation. When most of the cells shrinked to be round, and detached under gently shaking, a complete culture medium with twice the volume of trypsin was added quickly to terminate, and the cells were blown into single cells gently. The cell suspension was transferred to a centrifuge tube with appropriate size and centrifuged at 800 rpm for 5 minutes. The supernatant was discarded, and the cell clumps were resuspended with fresh complete culture medium, blown into single cells again, passaged and inoculated into new culture dishes (bottles) in a ratio of 1:3-1:6, the complete culture medium was supplemented, and then placed in a cell culture incubator at 37 °C and 5% CO₂ for culture.
3) Cell dosing treatment: The above-mentioned HeLa cells were digested, counted, and inoculated into each well of the 96 well cell culture plate (# 3988, Corning) at a density of 5000 cells/200 µl culture medium. They were cultured in a cell culture incubator at 37 °C and 5% CO₂ for 24 hours to allow the cells to fully adherent to the wall. Then, the original culture medium was replaced with the complete culture medium containing various gradient diluted (such as 100µM, 10µM, etc.) test compounds (each compound at each concentration with 3 repeated wells) as well as DMSO (# D5879, Sigma Aldrich) solvent control respectively, and they are cultured for additional 48-72 hours.
4) Preparation of test samples: The HeLa cells after 48-72 hours treatment with the above compounds were collected, centrifuged, and the supernatant culture medium was aspirated, washed twice with PBS which is pre-cooled in an ice bath, and the liquid was aspirated. 200µl of cell lysate (# P0013, Biyuntian) was added, and shaken vigorously for 30s and placed on ice for 5 minutes in triplicate. The cell lysis sample was centrifuged at 13000 rpm at 4 °C for 6 minutes. The supernatant was mixed with a 4 × Laemmli loading buffer (# 161-0747, Bio Rad) in 3: 1 volume to form a cell lysis protein sample, and then it was denatured in a metal bath at 100 °C for 6 minutes and used for Western blot analysis.
5) Detection of OCT4 protein biomarker: 4-15% pre-fabricated gradient gel (456-8084, Bio Rad) was installed into the electrophoresis tank, and the sufficient 1 × SDS-PAGE electrophoresis buffer was added. 20µLof the above cell lysis protein samples were added by 20µL pipette. The lid of the electrophoresis tank was putted on and the power supply was connected. First, electrophoresis is performed at a voltage of 80 V for about 30 minutes, and adjusted to 120 V when the bromophenol blue in the sample was compressed into a thin line at the boundary between the concentrated gel and the separation gel. The electrophoresis duration was adjusted according to the size of the target protein and internal reference protein bands. The pre-cooled 1 × membrane buffer solution was poured into a container with appropriate size, a "sandwich" structure of foam pad-filter paper-glue-PVDF membrane-filter paper-foam pad was assembled according to the instructions, and then placed into the electrophoresis tank. The ice cubes were added, the entire electrophoresis tank was placed in an ice bath, and the power supply was connected for membrane transfer, 250mA, 2h. PVDF membrane (IPVH00010, Millipore) was placed in 5% skimmed milk powder prepared by 1 × TBST and blocked at room temperature for 1 hour. They were then incubated with different primary antibodies (anti-OCT4A, # 2890S, CST; anti-GAPDH [HRP], # A00191-40, GenScript) and secondary antibodies (anti-Mouse IgG HRP-linked antibody, # 7076, CST; anti-Rabbit IgG HRP-linked antibody, # 7074, CST) sequentially (incubated at room temperature for 1 hour respectively) and washed (washed 3 times with 1 × TBST for 5 minutes each time). Finally, the PVDF film was placed in the middle of the plastic film, and ECL was added onto the film and reacted for 3 minutes, and then it was covered with another layer of plastic film, and exposed in the fully automatic chemiluminescence/fluorescence image analysis system (5200-Multi, Tianneng).

### 2. Test results

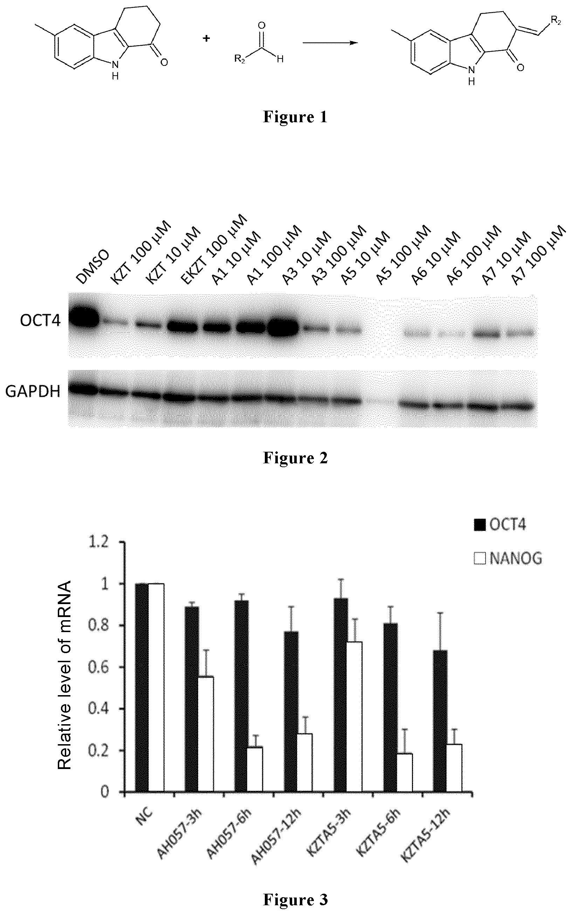

Figure 2 shows the inhibitory effect of the tested compound on the OCT4 protein in HeLa cells. As shown in Figure 2, each test compound shows different degrees of inhibitory effects on the OCT4 protein, wherein KZT-A5 shows the most significant effect in downregulating OCT4 protein. Moreover, compared with the KZT-A5 with concentration of 10µM, the KZT-A5 with 100µM shows more significant inhibitory effect on OCT4 protein, indicating that KZT-A5 can dose-dependently inhibit the expression of OCT4 protein in HeLa cells.

### (2) The inhibitory effect of compounds on the transcription of OCT4 target gene, NANOG, in tumor stem cells (CSC)

Test compounds: KZT-A5 (compound 5), AH057 (the structural formula of AH057 is shown in formula A, and AH057 is used to refer to this compound)

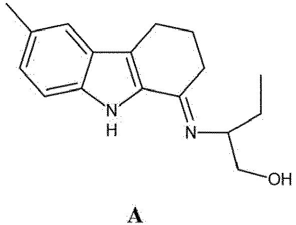

### 1. Test procedure

1) Cell source: HeLa cell line 3A11 with endogenous high expression of OCT4, prepared by the inventor's laboratory.
2) Cell culture and passage: The cell culture and passage method is the same as that described in "The inhibitory effect of compounds on the OCT4 protein in tumor stem cells (CSC)".
3) Cell dosing treatment: The cell dosing treatment method is the same as that described in "The inhibitory effect of compounds on the OCT4 protein in tumor stem cells (CSC)". Cervical cancer cells HeLa-3A11 were treated with DMSO (NC, blank control), 20 µM AH057, and 20 µM KZT-A5 for 3, 6, and 12 hours, respectively. After RNA was extracted from cell samples, the mRNA levels of the indicated genes were detected by fluorescence quantitative PCR (qRT PCR).

### 2. Test results

Figure 3 shows the inhibitory effect of KZT-A5 and control compound (AH057) on the transcription of the OCT4 target gene, NANOG, in HeLa cells, reflecting the inhibitory effect on the OCT4 protein. As shown in Figure 3, the mRNA levels of OCT4 and NANOG were downregulated to different degrees after 20µM KZT-A5 treatment for different times, this result suggests that KZT-A5 can inhibit the binding of OCT4 protein to the promoter regions of target genes such as NANOG by competitively disrupting the formation of OCT4/SOX2/DNA complexes, and thus inhibit their transcription. The inhibitory effect of KZT-A5 on NANOG gene transcription is similar to that of AH057, indicating that KZT-A5 may have the same target and action mechanism as AH057, i.e., it can inhibit the binding of OCT4 protein to target genes by competitively disrupting the formation of OCT4/SOX2/DNA complexes, and thus promote PSC and CSC differentiation. It is worth noting that after 6 hours of treatment, the mRNA level of NANOG was most significantly downregulated, and the ability of KZT-A5 to downregulate the mRNA level of NANOG was better than that of AH057 in cells treated for 6 and 12 hours.

### (3) The inhibitory effect of compounds on the JAK/STAT signaling pathway

### Test compounds: KZT-A5 (compound 5), AH057

### 1. Test procedure

1) Cell source: HeLa cell line 3A11 with endogenous high expression of OCT4, prepared by the inventor's laboratory.
2) Cell culture and passage: The cell culture and passage method is the same as that described in "The inhibitory effect of compounds on the OCT4 protein in tumor stem cells (CSC)".
3) Cell dosing treatment: The above-mentioned HeLa cells were digested, counted, and inoculated into each well of the 96 well cell culture plate (# 3988, Corning) at a density of 5000 cells/200µl culture medium. They were cultured in a cell culture incubator at 37 °C and 5% CO₂ for 24 hours to allow the cells to fully adherent to the wall. Then, the original culture medium was replaced with complete culture medium containing 1µM, 10µM, and 100µM KZT compounds or AH057 (each compound at each concentration with 3 repeated wells) as well as DMSO (# D5879, Sigma Aldrich) solvent control, and they are cultured for additional 72 hours.
4) Preparation of test samples: The preparation of test samples method is the same as that described in "The inhibitory effect of compounds on the OCT4 protein in tumor stem cells (CSC)".
5) Detection of JAK/STAT signaling pathway biomarker: HeLa cells have been reported to continuously activate JAK1/2 and further activate STAT3 through the IL-6 autocrine pathway. The detection method for signal pathway biomarkers is same as that described in the above "The inhibitory effect of compounds on the OCT4 protein in tumor stem cells (CSC)", wherein the antibody is: primary antibody (anti OCT4A, # 2890S, CST; anti AKT (Pan), # 4821, CST; Anti pAKT-S473, # 4060, CST; Anti STAT3, # 4904, CST; Anti pSTAT3-Y705, # 9145, CST; Anti GAPDH [HRP], # A00191-40, GenScript) and secondary antibodies (anti Mouse IgG HRP linked antibody, # 7076, CST; anti Rabbit IgG HRP linked antibody, # 7074, CST).

### 2. Test results

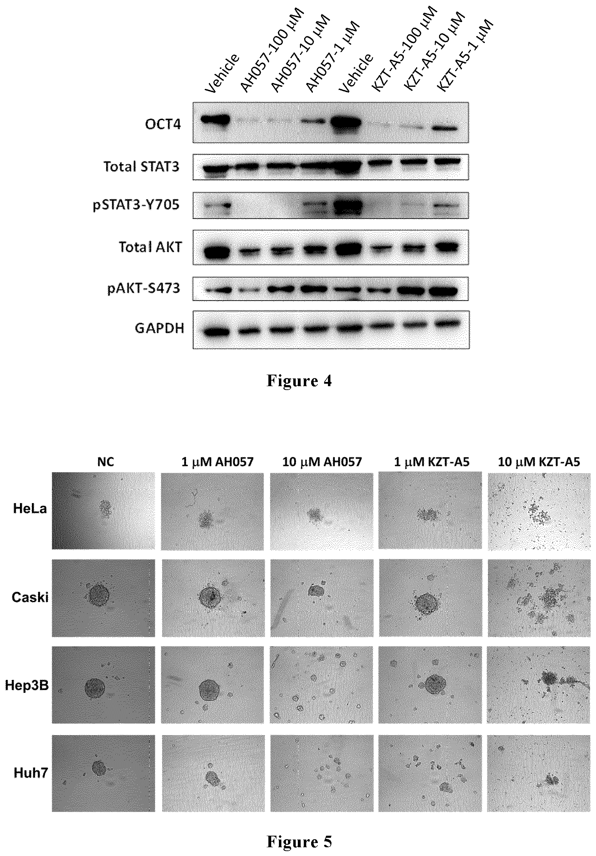

Figure 4 shows the inhibitory effect of KZT-A5 and control compound (AH057) on JAK/STAT signaling pathway biomarkers in HeLa cells, as well as the inhibitory effect on OCT4 protein. As shown in Figure 4, STAT3 activity is inhibited, indicating that KZT-A5 can effectively inhibit the activation of the JAK1/2-STAT3 signaling pathway in a dose-dependent manner. By inhibiting the kinase activity of JAK1/2, the activation of STAT3 is inhibited, thereby inhibiting the transcription of its target gene OCT4. Since STAT3 can partially mediate the transcription of stem genes such as OCT4 (Kim SY et al. Role of the IL-6-JAK1-STAT3-Oct-4 pathway in the conversion of non-stem cancer cells into cancer stem-like cells. Cell Signal. 2013, 25:961-9), KZT-A5 can synergistically block the transcription of stem genes such as OCT4 and NANOG through different pathways, and promote tumor stem cell differentiation and inhibit their proliferation.

The inhibitory effects of the above compounds on the OCT4 protein and JAK1/2-STAT3 signaling pathway in HeLa cells indicate that the series compounds of KZT can simultaneously target OCT4 protein and JAK1/2. On the one hand, they can inhibit the transcription of OCT4 and NANOG stem factors by blocking the formation of OCT4/SOX2/DNA complexes, and on the other hand, they can inhibit the downstream STAT3 transcriptional activity by inhibiting JAK1/2 kinase activity, which are a class of one-drug dual-target candidate drugs that simultaneously exert inhibitory effects on both OCT4 self-transcription and OCT4 transcriptional activity. This dual target inhibition mechanism can simultaneously target the inhibition and clearance of CSC and differentiated tumor cells, providing the possibility of completely blocking the bidirectional transformation between CSC and differentiated tumor cells, and having good potential application prospects in tumor eradication.

### Biological Test Example 2: In vitro pharmacodynamic test of compounds

### (1) The inhibitory effect of compounds on the generation of tumor microspheres by tumor cells

Test compounds: KZT-A5 (compound 5), AH057

### 1. Test procedure

1) Cell sources: cervical cancer cells HeLa and Caski, liver cancer cells Hep3B and Huh7 were all purchased from the Chinese Academy of Sciences cell bank (Shanghai).
2) Tumor microsphere culture: The specific culture method for tumor microspheres can be found in the literature (Cheng J et al. Tryptophan derivatives regulate the transfer of Oct4 in stem-like cancer cells. Nat Commun. 2015, 6:7209.). Each tumor cell was cultured separately in a medium containing 2% B27, 10 ng/ml BFGF, 5 ng/ml EGF, and 10 ng/ml LIF to form CSC rich tumor microspheres.
3) Cell dosing treatment: HeLa cell lines and Huh7 cell lines were inoculated in a 6-well plate containing the aforementioned culture medium at a density of 200 cells per well, while Hep3B cell lines and Caski cell lines were inoculated in a 6-well plate containing the aforementioned culture mediumat a density of 2000 cells per well. KZT-A5 or AH057 at concentrations of 1µM or 10µM was added, and the morphology of tumor microspheres was observed, photographed and recorded after 48 hours of treatment.

### 2. Test results

Figure 5 shows the cellular morphological characterization after 48 hours of KZT-A5 or control compound (AH057) treatment on tumor microspheres. As shown in Figure 5, tumor microspheres treated with KZT-A5 shows scattered distribution, and compared with the 1 µL administration concentration of KZT-A5, the 10 µL administration concentration of KZT-A5 has a better inhibitory effect on the generation of tumor microspheres, indicating that KZT-A5 can dose-dependently inhibit the generation of tumor microspheres, and confirmed its ability to promote CSC differentiation and/or inhibit CSC proliferation. According to the test results of the tumor microsphere system, it can be seen that KZT-A5 can promote the differentiation and apoptosis of CSC and tumor cells, inhibit proliferation , invasion and migration of tumor cell.

By comparing the effects of AH057 and KZT-A5 on tumor microspheres cultured in Huh7 cells, it can be seen that 1µM KZT-A5 shows more significant inhibitory effect compared with the same concentration of AH057, indicating that KZT-A5 has the potential to have better pharmacological effects in inhibiting the proliferation and differentiation of liver cancer cells than AH057.

By comparing the effects of AH057 and KZT-A5 on tumor microspheres cultured in HeLa and Caski cells, it can be seen that when the concentration of KZT-A5 reaches to 10µM, its inhibitory effect on tumor microspheres cultured in cervical cancer cells is more significant than that of the control compound AH057.

### (2) The inhibitory effect of compounds on human tumor cell lines from different tissue sources (1)

Test compound: KZT-A5 (compound 5)

### 1. Test procedure

1) Cell sources: HeLa (human cervical cancer cells), CaSki (human cervical cancer intestinal metastasis cells), Huh7 (human liver cancer cells), HepG2 (human liver cancer cells), Hep3B (human liver cancer cells) were purchased from the Chinese Academy of Sciences cell bank (Shanghai); LO2 (human normal liver cells) and HcerEpic (human normal uterine epithelial cells) were from ATCC.
2) Cell culture and passage: The cell culture and passage method is the same as that described in "The inhibitory effect of compounds on the OCT4 protein in tumor stem cells (CSC)".
3) Cell dosing treatment: The each type of cells mentioned above was digested, counted, and inoculated in each well of the 96 well cell culture plate (# 3988, Corning) at a density of 5000 cells/200µl culture medium. They were cultured in a cell culture incubator at 37 °C and 5% CO₂ for 24 hours to allow the cells to fully adherent to the wall. Then, the original culture medium was replaced with complete culture medium containing gradient diluted KZT-A5 (each concentration with 3 repeated wells) as well as DMSO (# D5879, Sigma Aldrich) solvent control, and they are cultured for additional 72 hours.
4) Pharmacodynamic test and statistics: First, the morphology of cells treated with the above compounds was observed, photographed and recorded using an inverted phase contrast microscope (X71, Olympus). Then the original culture medium was replaced with phenol red free culture medium containing CCK-8 detection reagent (# E606335, Shanghai Shenggong) respectively, and they are cultured in the incubator for additional 2 hours. Then the OD450nm absorbance value (OD value) was measured on a multifunctional enzyme-linked immunosorbent assay (168-1130, Bio Rad). After the cells were treated with the test compound, the equations for calculating the cell survival rate or cell growth rate is: survival rate=OD value of the treated group/OD value of the control group ×100%; the equations for calculating the growth inhibition rate of compounds on cell proliferation is: inhibition rate=(OD value of control group -OD value of the treated group)/OD value of control group×100%. Further, the IC₅₀ of each compound was calculated in SPSS based on the inhibition rate. The IC₅₀ value data of the compound on each type of tumor cells is the average of three wells in one test.

### 2. Test results

**Table 2: Inhibition activity of KZT-A5 on proliferation of multiple human tumor cell lines in vitro**

| Cell line | IC₅₀ (µM) |
|---|---|
| HeLa (human cervical cancer cells) | 0.57 |
| CaSki (human cervical cancer intestinal metastatic cells) | 6.38 |
| Huh7 (human liver cancer cells) | 0.80 |
| HepG2 (human liver cancer cells) | 3.13 |
| Hep3B (human liver cancer cells) | 36.56 |
| LO2 (human normal liver cells) | 82.08 |
| HcerEpic (human normal uterine epithelial cells) | >100 |

It can be seen from Table 2 that the IC₅₀ values of KZT-A5 on Huh7 cell lines in human liver cancer cells and HeLa cell lines in human cervical cancer cells are relatively low, both lower than1µM, indicating it has superior ability to inhibit the proliferation of liver cancer cells and cervical cancer cells. In addition, IC₅₀ values of compounds 8, 14, 16, 18, 19, 20, 21, 22, 23, 24, 25, 27, 28, 31, 32, 33, 34, 35, 36, 37, and 38 on HeLa cell lines in human cervical cancer cells are much less than 50µM.

### (3) The inhibitory effect of compounds on human tumor cell lines from different tissue sources (2)

Test compounds: KZT-A5 (compound 5), AH057

### 1. Test procedure

1) Cell sources: Huh7 (human liver cancer cells), HepG2 (human liver cancer cells), Hep3B (human liver cancer cells), and HeLa (human cervical cancer cells) were all purchased from the Chinese Academy of Sciences cell bank (Shanghai), while LO2 (human normal liver cells) was obtained from ATCC.
2) Cell culture and passage: The cell culture and passage method is the same as that described in "The inhibitory effect of compounds on human tumor cell lines from different tissue sources (1)".
3) Cell dosing treatment: The cell dosing treatment method is the same as that described in "The inhibitory effect of compounds on human tumor cell lines from different tissue sources (1).
4) Pharmacodynamic test and statistics: The pharmacodynamic test and statistics method is the same as that described in "The inhibitory effect of compounds on human tumor cell lines from different tissue sources (1)". The IC₅₀ value data of the compound on each type of tumor cells is the average ± standard deviation of three wells in one test.

### 2. Test results

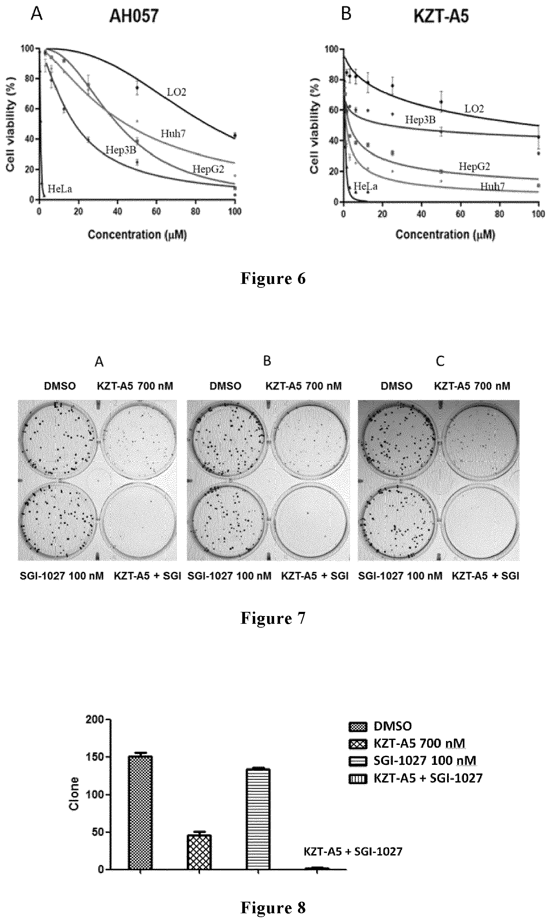

Figure 6 shows the effects of KZT-A5 and AH057 on the viability of different tumor cells. As shown in Figure 6, compared with AH057, KZT-A5 has a more significant inhibitory effect on the viability of Huh7 and HepG2 cells in liver cancer cells.

**Table 3 anti-tumor viability of KZT-A5 and AH057 on human tumor cells in vitro**

| Cell line | IC₅₀ (µM) | | |
|---|---|---|---|
| | KZT-A5 | AH057 | P-value |
| Huh7 (human liver cancer cells) | 1.42 ± 0.12 | 44.21 ± 1.61 | 0.0004 |
| HepG2 (human liver cancer cells) | 3.16 ± 0.08 | 40.02 ± 0.08 | 0.0017 |
| Hep3B (human liver cancer cells) | 23.52 ± 1.51 | 18.02 ± 1.20 | 0.0090 |
| LO2 (human normal liver cells) | 96.52 ± 13.04 | 85.53 ± 2.92 | 0.279 |
| HeLa (human cervical cancer cells) | 0.70 ± 0.01 | 0.63 ± 0.03 | 0.026 |

According to Table 3, AH057 was used as a control to compared in vitro efficacy of KZT-A5 and AH057 on human liver cancer cells and cervical cancer cells. It can be seen that the inhibitory effect of KZT-A5 on Hep3B and HeLa cell lines is comparable to AH057. Compared with AH057, KZT-A5 has lower IC₅₀ values and high significance on Huh7 and HepG2 cell lines of liver cancer cells. This result confirmed that KZT-A5 has a significantly better inhibitory effect on liver cancer cell proliferation than AH057, and has a better in vitro efficacy.

### Biological Test Example 3: KZT-A5 and SGI-1027 synergistically inhibit in vitro proliferation of HeLa cells

Test compound: KZT-A5

SGI-1027 is an inhibitor of DNA methyltransferase II, with CAS number 1020149-73-8. Bliss independent model was used to screen and determine SGI-1027 as the compound for combination with KZT-A5, and the screening process was carried out according to the methods in the following literature: Goldoni M, Johansson C. A mathematical approach to study combined effects of toxins in vitro: evaluation of the Bliss independence criterion and the Loewe additivity model Toxic In Vitro 2007, 21:759-69.

### 1. Test procedure

1) Cell source: HeLa cells were purchased from the Chinese Academy of Sciences Cell Bank (Shanghai).
2) Cell culture and passage: The cell culture and passage method is the same as that described in "Inhibition of OCT4 protein in tumor stem cells (CSCs) by compounds".
3) Cell dosing treatment: The celles were inoculated into a 6-well plate containing the aforementioned culture medium at a density of 200 cells per well. They were divided into four groups, and DMSO (as blank control group), 700nM KZT-A5 (as KZT-A5 single drug group), 100nM SGI-1027 (as SGI-1027 single drug group), and 700nM KZT-A5+100nM SGI-1027 (as combination group) was added respectively. After two weeks, they were photographed, and the number of clones was counted in each group respectively.

### 2. Test results

Figure 7 shows the inhibitory effects of blank control group, KZT-A5 single drug group, SGI-1027 single drug group, and combination group on HeLa cell proliferation. Figures 7A, 7B, and 7C respectively represent the cell proliferation after three technical repetitions. As shown in Figure 7, compared with the DMSO control group, SGI-1027 single drug only slightly inhibits clone formation, KZT-A5 single drug significantly inhibits clone formation, and the combination of KZT-A5 and SGI-1027 almost completely inhibits clone formation.

Figure 8 shows the statistical results of the average number of clones obtained from three technical repetitions, and the statistical graph of the cell clone count after treatment of DMSO, KZT-A5 single drug, SGI-1027 single drug, and KZT-A5+SGI-1027 combination. According to the statistical results in Figure 8, the number of clones formed by the combination of KZT-A5 and SGI-1027 is much lower than that formed by KZT-A5 or SGI-1027 single drug. This is due to the fact that KZT-A5 and SGI-1027 can act joinly, and simultaneously inhibit the JAK/STAT/OCT4 and DNMT1 pathways, thus they have a strong inhibitory effect on tumor cell proliferation, and inducing cell cycle arrest and apoptosis. Compounds such as KZT-A5 and SGI-1027 have a highly effective synergistic effect in inhibiting tumor cell proliferation. It can be seen that the combination of KZT-A5 and DNMT inhibitors provides a new approach for combination therapy of simultaneously blocking multiple interacting signaling/survival pathways, and it can achieve therapeutic effects with reduced dosage, thereby minimizing the toxicity and other side effects of high-dose single drugs, reducing the possibility of drug resistance and overlapped toxicity at the same time, has good development prospects.

The present invention explores the action mechanism of tetrahydrocarbazole compounds at the molecular level and verifies their dual target inhibitory effect on tumor stem cells (CSCs), which can simultaneously target on promoting CSC differentiation and killing differentiated tumor cells, providing the possibility of completely blocking the bidirectional transformation between CSC and differentiated tumor cells, and has good potential application prospects in eradicating tumors. The tetrahydrocarbazole compounds provided in the present invention have significant inhibitory effects on tumors such as cervical cancer and liver cancer, wherein the inhibitory effect against liver cancer is more significant, and compared with other control substances, the inhibitory effect of the compounds provided in the present invention on liver cancer is very prominent and unexpected, and have very good research and application prospects in the field of liver cancer treatment.

All references mentioned in the present invention are cited as references in this application, just as each reference is cited separately. In addition, it should be understood that after reading the above teaching content of the present invention, those skilled in the art can make various changes or modifications to the present invention, and these equivalent forms also fall within the scope of the appended claims of the present application.

## Claims

1. A compound as shown in formula (I), an optical isomer or a pharmaceutically acceptable salt thereof: 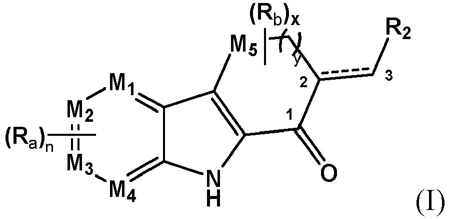 wherein, 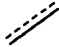
is a single or double bond;
R₂ is selected from the group consisting of phenyl, 5-7-membered heteroaryl, 8-12-membered fused heteroaryl, which is unsubstituted or substituted by one or more R_{c};
M₁, M₂, M₃ and M₄ are independently selected from the group consisting of CH, CDand N; and when M₁, M₂, M₃ or M₄ is CH, the hydrogen atoms on the CH can be optionally substituted by Rₐ;
M₅ is selected from the group consisting of CH₂, CHD, CD₂, NH, S, S(=O), S(=O)₂, and S(=O)(=NH); and when M₅ is CH₂ or NH, the hydrogen atom on the CH₂ or NH can be optionally substituted by R_{b};
Rₐ is selected from the group consisting of hydrogen, deuterium, halogen, hydroxyl, carboxyl, amino, cyano, sulfonyl, C(O)NH₂, C₁₋₅ alkyl, C₂₋₅ alkenyl, C₂₋₅ alkynyl, C₁₋₅ alkoxy, C₁₋₅ haloalkyl, C₂₋₅ haloalkenyl, C₂₋₅ haloalkynyl, C₁₋₅ haloalkoxy, C₂₋₅ ester group, C₁₋₅ carbonyl, C(O)NH(C₁₋₅alkyl), C₃₋₈ saturated or partially unsaturated carbocyclyl, 3-10 membered saturated or partially unsaturated heterocyclyl, C₆₋₁₀ aryl, and 5-12 membered heteroaryl; and the alkyl, alkenyl, alkynyl, alkoxy, haloalkyl, haloalkenyl, haloalkynyl, haloalkoxy, ester group, carbonyl, carbocyclyl, heterocyclyl, aryl and heteroaryl are independently and optionally substituted by one or more substituents selected from the group consisting of halogen, deuterium, hydroxyl, carboxyl, amino, nitro, cyano, sulfonyl, C₁₋₃alkyl and C₁₋₃ amino;
R_{b} is selected from the group consisting of hydrogen, deuterium, halogen, hydroxyl, carboxyl, cyano, sulfonyl, C₁₋₅ alkyl, C₂₋₅ alkenyl, C₂₋₅ alkynyl, C₁₋₅ alkoxy, C₁₋₅ haloalkyl, C₂₋₅ haloalkenyl, C₂₋₅ haloalkynyl, C₁₋₅ haloalkoxy, C₂₋₅ ester group, C₁₋₅ carbonyl, C₃₋₈ saturated or partially unsaturated carbocyclyl, 3-10 memberedsaturated or partially unsaturated heterocyclyl, C₆₋₁₀ aryl, and 5-12 membered heteroaryl; and the alkyl, alkenyl, alkynyl, alkoxy, haloalkyl, haloalkenyl, haloalkynyl, haloalkoxy, ester group, carbonyl, carbocyclyl, heterocyclyl, aryl and heteroaryl are independently and optionally substituted by one or more substituents selected from the group consisting of halogen, deuterium, hydroxyl, carboxyl, nitro, cyano, sulfonyl, C₁₋₃ alkyl and C₁₋₃ amino;
R_{c} is selected from the group consisting of hydrogen, deuterium, halogen, hydroxyl, carboxyl, amino, nitro, cyano, sulfonyl, C₁₋₅ alkyl, C₂₋₅ alkenyl, C₂₋₅ alkynyl, C₁₋₅ alkoxy, C₁₋₅ haloalkyl, C₂₋₅ haloalkenyl, C₂₋₅ haloalkynyl, C₁₋₅ haloalkoxy, C₁₋₅ amino, C₂₋₅ ester group, C₁₋₅ carbonyl, C₃₋₈ saturated or partially unsaturated carbocyclyl, 3-10 membered saturated or partially unsaturated heterocyclyl, C₆₋₁₀ aryl, and 5-12 membered heteroaryl; and the alkyl, alkenyl, alkynyl, alkoxy, haloalkyl, haloalkenyl, haloalkynyl, haloalkoxy, amino, ester group, carbonyl, carbocyclyl, heterocyclyl, aryl and heteroaryl are independently and optionally substituted by one or more substituents selected from the group consisting of halogen, deuterium, hydroxyl, carboxyl, amino, nitro, cyan, sulfonyl and C₁₋₃ alkyl;
N is 0, 1, 2 or 3;
X is 0, 1, 2 or 3; and
Y is 0, 1 or 2.
Preferably, the compound does not include compounds selected from the group consisting of:

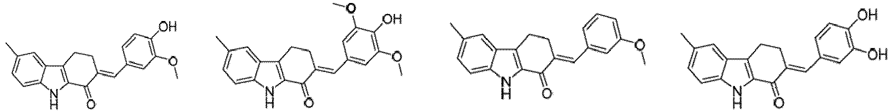

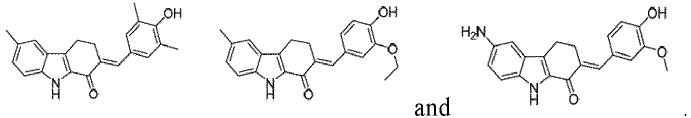

2. The compound according to claim 1, an optical isomer or a pharmaceutically acceptable salt thereof, wherein, the compound has a structure as shown in formula (II) or formula (III): 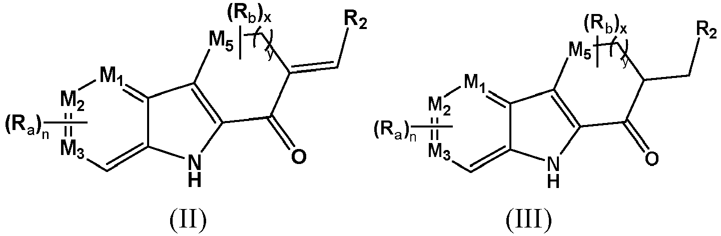
wherein, R₂, M₁, M₅, Rₐ, R_{b}, n, x and y are defined as described in claim 1.
In another preferred embodiment, the compound has a structure selected from the group consisting of: 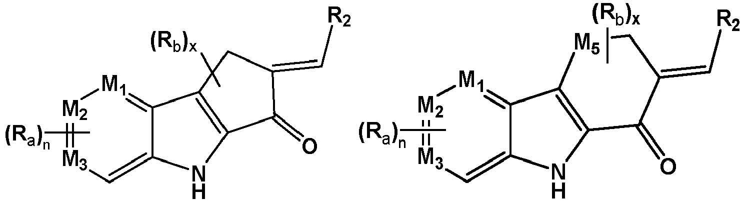 and 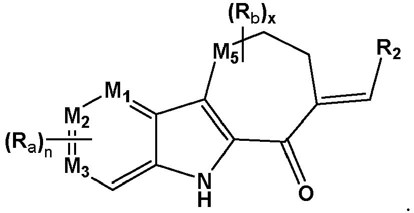
In another preferred embodiment, the compound has a structure selected from the group consisting of:

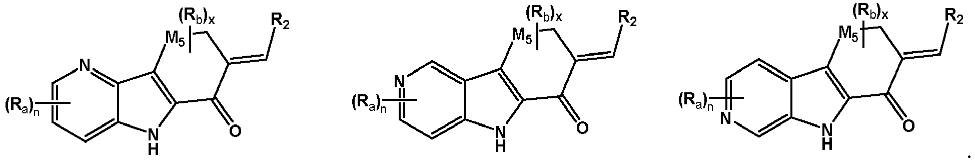

3. The compound according to claim 1, an optical isomer or a pharmaceutically acceptable salt thereof, wherein, R₂ is a unsubstituted or one or more R_{c} substituted phenyl, or a unsubstituted or one or more R_{c} substituted 5-10 membered heteroaryl;
R_{c} is selected from the group consisting of hydrogen, halogen, hydroxyl, carboxyl, amino, nitro, cyano, sulfonyl, C₁₋₅ alkyl, C₂₋₅ alkenyl, C₂₋₅ alkynyl, C₁₋₅ alkoxy, C₁₋₅ haloalkyl, C₂₋₅ haloalkenyl, C₂₋₅ haloalkynyl, C₁₋₅ haloalkoxy, C₁₋₅ amino, and C₂₋₅ ester group.
In another preferred embodiment, R₂ is selected from the group consisting of phenyl,

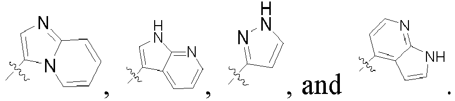

4. The compound according to claim 1, an optical isomer or a pharmaceutically acceptable salt thereof, wherein, R_{b} is selected from the group consisting of hydrogen, halogen, hydroxyl, carboxyl, cyano, sulfonyl, C₁₋₄alkyl, C₁₋₄ alkoxy, C₁₋₄ haloalkyl, C₁₋₄ haloalkoxy, C₂₋₄ ester group and C₁₋₄ carbonyl; and the alkyl, alkoxy, haloalkyl, haloalkoxy, ester group and carbonyl are independently and optionally substituted by one or more substituents selected from the group consisting of halogen, hydroxyl, carboxyl, nitro, cyano, sulfonyl, C₁₋₃ alkyl and C₁₋₃ amino.

5. The compound according to claim 1, an optical isomer or a pharmaceutically acceptable salt thereof, wherein, Rₐ is selected from the group consisting of hydrogen, halogen, hydroxyl, carboxyl, cyano, C₁₋₄ alkyl, C₂₋₄ alkenyl, C₂₋₄ alkynyl, C₁₋₄ alkoxy, C₂₋₄ ester group, C₁₋₄ carbonyl, 3-8-membered saturated or partially unsaturated heterocyclyl, C₆₋₁₀aryl, 5-12membered heteroaryl; and the alkyl, alkenyl, alkynyl, alkoxy, ester group, carbonyl, heterocyclyl, aryl and heteroaryl are independently and optionally substituted by one or more substituents selected from the group consisting of halogen, hydroxyl, carboxyl, amino, nitro, cyano, sulfonyl, C₁₋₃ alkyl and C₁₋₃ amino.

6. A compound as shown in formula (IA), an optical isomer or a pharmaceutically acceptable salt thereof: 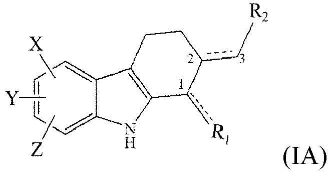 wherein,
R₁ is hydroxyl and is connected to the carbon atom at position 1 via a single bond, or R₁ is an oxygen atom or -N(CH₂)ₙOH and is connected to the carbon atom at position 1 via a double bond, wherein, n is 1 or 2;
the carbon atom at position 2 is connected to the carbon atom at position 3via a single or double bond; R₂ is selected from any one of the group consisting of unsubstituted or substituted monocyclyl or monoheterocyclyl, unsubstituted or substituted fused-cyclyl or fused heterocyclyl;
X, Y and Z are independently selected from any one of the group consisting of hydrogen, halogen, hydroxyl, carboxyl, amino, nitro, cyano, sulfonyl, C₁₋₅ alkyl, C₁₋₅ alkoxy, C₁₋₅ haloalkyl and C₁₋₅ haloalkoxy.

7. The compound according to claim 6, an optical isomer or a pharmaceutically acceptable salt thereof, wherein, in the compound shown in formula I, R₁ is an oxygen atom and is connected to the carbon atom at position1 via a carbon-oxygen double bond; the carbon atom at position 2 is connected to the carbon atom at position 3 via a carbon-carbon double bond, and comprises the compound shown in formula (IIA): 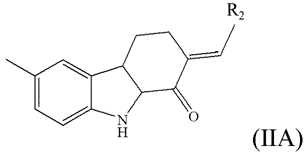 in formula IIA:
R₂ is selected from any one of group consisting of unsubstituted or substituted monocyclyl or monoheterocyclyl, unsubstituted or substituted fused-cyclyl or fused heterocyclyl; and the substituents on the monocyclyl, monoheterocyclyl, fused-cyclyl or fused heterocyclyl are selected from one or more group consisting of halogen, hydroxyl, carboxyl, amino, nitro, cyano, sulfonyl, C₁₋₅ alkyl, C₁₋₅ alkoxy, C₁₋₅ haloalkyl and C₁₋₅ haloalkoxy.

8. The compound according to claim 7, an optical isomer or a pharmaceutically acceptable salt thereof, wherein, in the compound shown in formula IIA, R₂ is a phenyl, and at least one hydrogen atom on the phenyl is substituted by hydroxyl.

9. The compound according to claim 1, an optical isomer or a pharmaceutically acceptable salt thereof, wherein, the compound is selected from the group consisting of:

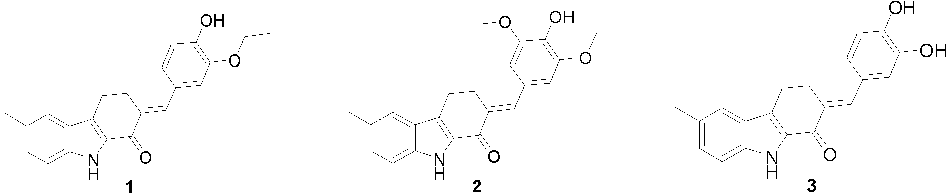

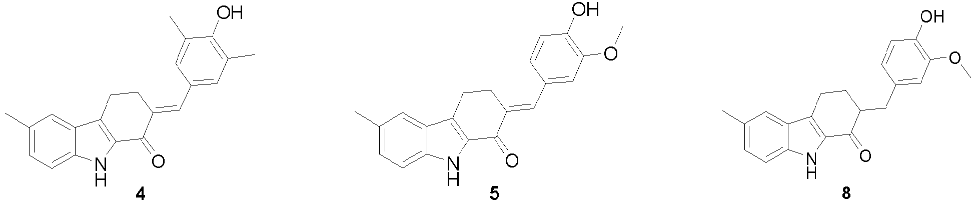

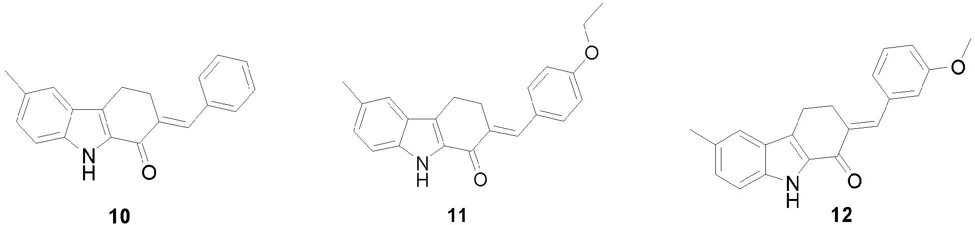

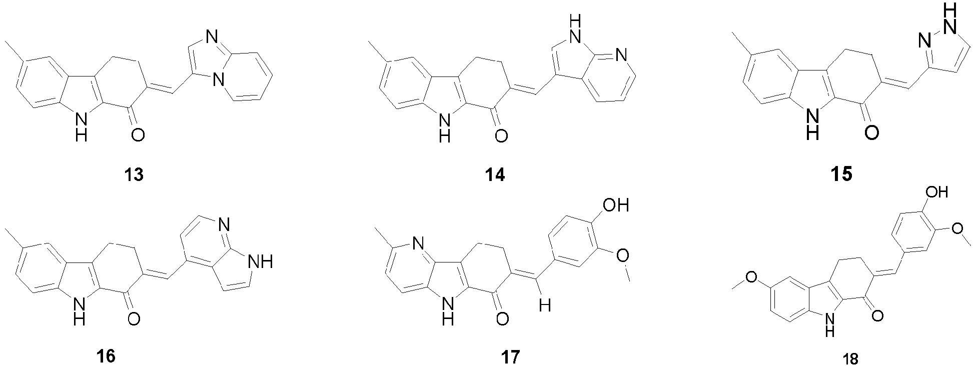

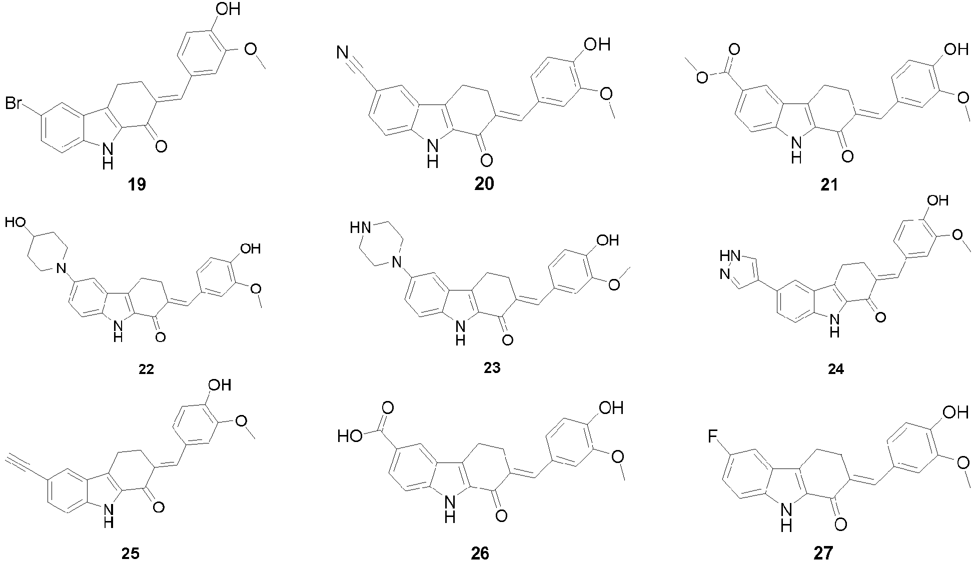

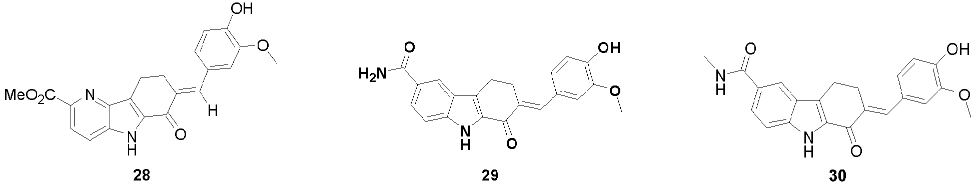

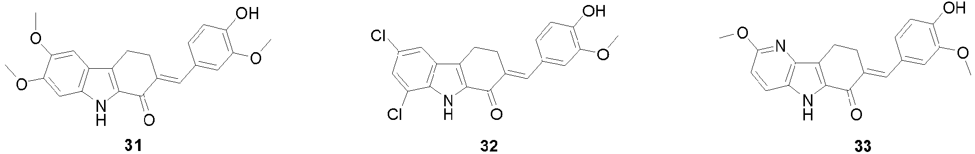

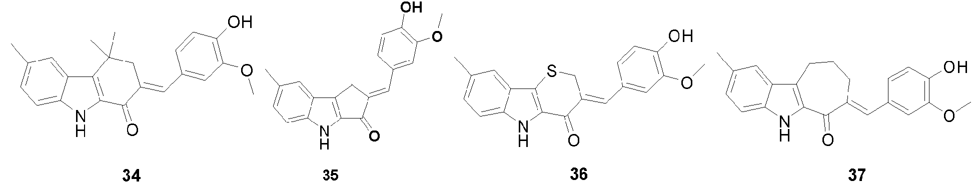

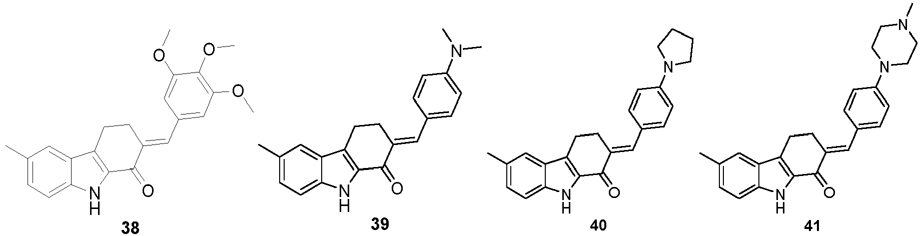

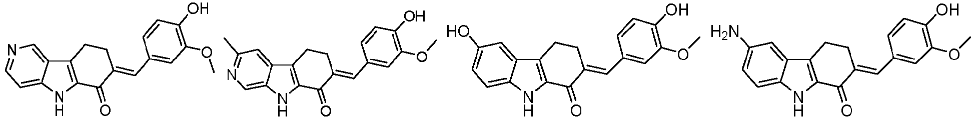

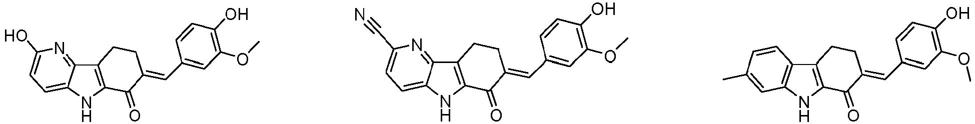

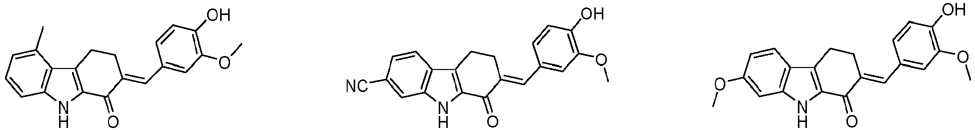

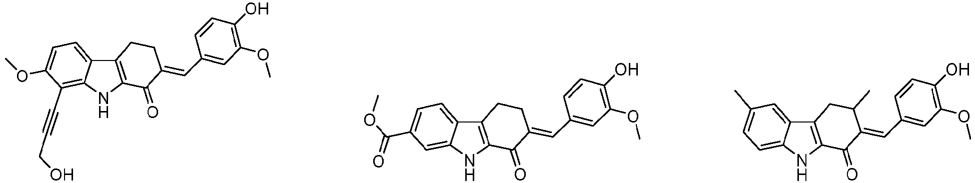

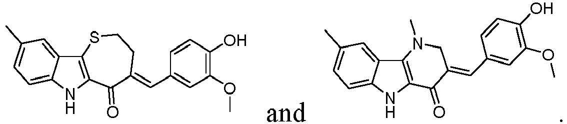

10. A pharmaceutical composition, comprising (1) a compound according to any one of claims 1-9, an optical isomer or a pharmaceutically acceptable salt thereof; and optional (2) pharmaceutically acceptable carriers, excipients or other active drugs.

11. The pharmaceutical composition according to claim 10, wherein, the pharmaceutical composition further comprises a second therapeutic component, and the second therapeutic component is a DNA methyltransferase inhibitor; preferably, the DNA methyltransferase inhibitor is SGI-1027.

12. A use of a compound according to any one of claims 1-9, an optical isomer or a pharmaceutically acceptable salt thereof, or a pharmaceutical composition according to claim 10 in the preparation of a drug for treating and/or preventing cancers.

13. The use according to claim 12, wherein, the cancers are selected from the group consisting of liver cancer, lung cancer, breast cancer, pancreatic cancer, gastric cancer, cervical cancer, ovarian cancer, head and neck cancer.

14. The use according to claim 12, wherein, the treating and/or preventing cancers comprises: administrating the compound as shown in formula I according to any one of claims 1-9, an optical isomer or a pharmaceutically acceptable salt thereof, or the pharmaceutical combination according to claim 7 in combination with a DNA methyltransferase inhibitor.

15. The use according to claim 14, wherein, the DNA methyltransferase inhibitor is SGI-1027.
